# EUROPEAN PATENT SPECIFICATION

(11) **EP 2 614 078 B1**
(45) Date of publication and mention of the grant of the patent: **07.11.2018**
(21) Application number: 11758431.8
(22) Date of filing: 08.09.2011
(51) Int. Cl.: C07K 14/525, C12N 5/0775, C07K 14/705, A61K 38/17

(54) **STEM CELL CULTURE MEDIA AND METHODS**
STAMMZELLENKULTURMEDIUM UND VERFAHREN
MILIEU DE CULTURE DE CELLULE SOUCHE ET PROCEDE

(30) Priority: 10.09.2010 EP 10382244
(43) Date of publication of application: 17.07.2013
(73) Proprietor: TiGenix, S.A.U., 28760 Tres Cantos (Madrid) (ES); Consejo Superior De Investigaciones Científicas (CSIC), 28006 Madrid (ES)
(72) Inventor: LOMBARDO, Eleuterio, 28760 Tres Cantos - Madrid (ES); PLANELLES CARAZO, Lourdes, 28006 Madrid (ES); ZONCA, Manuela, 28006 Madrid (ES)
(74) Representative: ABG Intellectual Property, S.L.
(86) International application number: PCT/EP2011/065540
(87) International publication number: WO 2012/032112

(56) References cited:
- WO-A2-2007/016366
- BOSSEN C ET AL: "BAFF, APRIL and their receptors: Structure, function and signaling", SEMINARS IN IMMUNOLOGY, W.B. SAUNDERS COMPANY, PA, US, vol. 18, no. 5, 1 October 2006 (2006-10-01), pages 263-275, XP024908025, ISSN: 1044-5323, DOI: 10.1016/J.SMIM.2006.04.006 [retrieved on 2006-10-01]
- MACKAY FABIENNE ET AL: "Cracking the BAFF code", NATURE REVIEWS IMMUNOLOGY, vol. 9, no. 7, July 2009 (2009-07), pages 491-502, XP9154275,
- MOISINI I ET AL: "BAFF: a local and systemic target in autoimmune diseases", CLINICAL AND EXPERIMENTAL IMMUNOLOGY, vol. 158, no. 2, November 2009 (2009-11), pages 155-163, XP9154278, ISSN: 0009-9104
- ALEXAKI VASSILIA-ISMINI ET AL: "Adipocytes as Immune Cells: Differential Expression of TWEAK, BAFF, and APRIL and Their Receptors (Fn14, BAFF-R, TACI, and BCMA) at Different Stages of Normal and Pathological Adipose Tissue Development", JOURNAL OF IMMUNOLOGY, vol. 183, no. 9, November 2009 (2009-11), pages 5948-5956, XP9154273, ISSN: 0022-1767

### SEQUENCE LISTING

<110> CELLERIX S.A. CONSEJO SUPERIOR DE INVESTIGACIONES CIENTÍFICAS (CSIC)
<120> STEM CELL CULTURE MEDIA AND METHODS
<130> P6263PC00
<150> EP 10382244.1
   <151> 2010-09-10
<160> 16
<170> PatentIn version 3.5
<210> 1
   <211> 285
   <212> PRT
   <213> H.sapiens
<400> 1
   Met Asp Asp Ser Thr Glu Arg Glu Gln Ser Arg Leu Thr Ser Cys Leu
   1               5                   10                  15
   Lys Lys Arg Glu Glu Met Lys Leu Lys Glu Cys Val Ser Ile Leu Pro
               20                  25                  30
   Arg Lys Glu Ser Pro Ser Val Arg Ser Ser Lys Asp Gly Lys Leu Leu
           35                  40                  45
   Ala Ala Thr Leu Leu Leu Ala Leu Leu Ser Cys Cys Leu Thr Val Val
       50                  55                  60
   Ser Phe Tyr Gln Val Ala Ala Leu Gln Gly Asp Leu Ala Ser Leu Arg
   65                  70                  75                  80
   Ala Glu Leu Gln Gly His His Ala Glu Lys Leu Pro Ala Gly Ala Gly
                   85                  90                  95
   Ala Pro Lys Ala Gly Leu Glu Glu Ala Pro Ala Val Thr Ala Gly Leu
               100                 105                 110
   Lys Ile Phe Glu Pro Pro Ala Pro Gly Glu Gly Asn Ser Ser Gln Asn
           115                 120                 125
   Ser Arg Asn Lys Arg Ala Val Gln Gly Pro Glu Glu Thr Val Thr Gln
       130                 135                 140
   Asp Cys Leu Gln Leu Ile Ala Asp Ser Glu Thr Pro Thr Ile Gln Lys
   145                 150                 155                 160
   Gly Ser Tyr Thr Phe Val Pro Trp Leu Leu Ser Phe Lys Arg Gly Ser
                   165                 170                 175
   Ala Leu Glu Glu Lys Glu Asn Lys Ile Leu Val Lys Glu Thr Gly Tyr
               180                 185                 190
   Phe Phe Ile Tyr Gly Gln Val Leu Tyr Thr Asp Lys Thr Tyr Ala Met
           195                 200                 205
   Gly His Leu Ile Gln Arg Lys Lys Val His Val Phe Gly Asp Glu Leu
       210                 215                 220
   Ser Leu Val Thr Leu Phe Arg Cys Ile Gln Asn Met Pro Glu Thr Leu
   225                 230                 235                 240
   Pro Asn Asn Ser Cys Tyr Ser Ala Gly Ile Ala Lys Leu Glu Glu Gly
                   245                 250                 255
   Asp Glu Leu Gln Leu Ala Ile Pro Arg Glu Asn Ala Gln Ile Ser Leu
               260                 265                 270
   Asp Gly Asp Val Thr Phe Phe Gly Ala Leu Lys Leu Leu
           275                 280                 285
<210> 2
   <211> 250
   <212> PRT
   <213> H.sapiens
<400> 2
<210> 3
   <211> 184
   <212> PRT
   <213> H.sapiens
<400> 3
<210> 4
   <211> 293
   <212> PRT
   <213> H.sapiens
<400> 4
<210> 5
   <211> 20
   <212> DNA
   <213> Artificial
<220>
   <223> Primer
<400> 5
   aagggtatcc ctggcagagt
<210> 6
   <211> 19
   <212> DNA
   <213> Artificial
<220>
   <223> Primer
<400> 6
   gcaggacaga gtgctgctt 19
<210> 7
   <211> 23
   <212> DNA
   <213> Artificial
<220>
   <223> Primer
<400> 7
   actgaaaatc tttgaaccac cag 23
<210> 8
   <211> 21
   <212> DNA
   <213> Artificial
<220>
   <223> Primer
<400> 8
   ttgcaagcag tcttgagtga c 21
<210> 9
   <211> 24
   <212> DNA
   <213> Primer
<400> 9
   caactcggga aggtaccaag gatt 24
<210> 10
   <211> 24
   <212> DNA
   <213> Artificial
<220>
   <223> Primer
<400> 10
   cgccaccagg aagcagcaga ggac 24
<210> 11
   <211> 24
   <212> DNA
   <213> Artificial
<220>
   <223> Primer
<400> 11
   ttttcgtgct aatgtttttg ctaa 24
<210> 12
   <211> 24
   <212> DNA
   <213> Artificial
<220>
   <223> Primer
<400> 12
   ttcatcacca gtcctgctct tttc 24
<210> 13
   <211> 18
   <212> DNA
   <213> Artificial
<220>
   <223> Primer
<400> 13
   ctggtcctgg tgggtctg 18
<210> 14
   <211> 18
   <212> DNA
   <213> Artificial
<220>
   <223> Primer
<400> 14
   accttgtcca ggggctct 18
<210> 15
   <211> 20
   <212> DNA
   <213> Artificial
<220>
   <223> Primer
<400> 15
   cccagcacaa tgaagatcaa 20
<210> 16
   <211> 20
   <212> DNA
   <213> Artificial
<220>
   <223> Primer
<400> 16
   cgatccacac ggagtacttg 20

## Description

### FIELD OF THE INVENTION

The present invention provides novel uses of stem cell culture media and methods, in particular uses of culture media and methods for expanding populations of mesenchymal stem cells.

### BACKGROUND

There is great interest in culture media and methods for expanding populations of multipotent stem cells, particularly adipose-derived mesenchymal stem cells (ASC cells). Clinical applications of multipotent stem cells require reproducible cell culture methods to provide adequate numbers of cells of suitable quality. Numerous different culture media and methods have been tested for multipotent stem cells, with varying degrees of success.

It is widely recognised that there remains a great need for further improvements to multipotent stem cell culture media and methods, in particular for improvements to ASC cell culture media and methods.

Tumor necrosis factor (TNF)-like receptors are members of a superfamily of proteins that are key in the regulation of the maturation, survival and apoptosis of immune cells. Two members of this superfamily B-cell maturation protein A (BCMA; TNFRSF17; BCM) and transmembrane activator and CAML interactor (TACI; TNFRSF13B) share the ability to bind to two TNF ligands B-cell activating factor (BAFF; synonyms BLyS, THANK, TALL-1, and zTNF4, TNFSF13B) and a proliferation-inducing ligand (APRIL; synonyms TALL-2 and TRDL-1, TNFSF13A). Both are cell membrane single pass type III proteins expressed by cells and associated with humoral immunity.

BCMA (Tumor necrosis factor receptor superfamily member 17; CD269; B-cell maturation protein) is expressed primarily on mature B-cells and other B-cell lines and plays an important role in B cell development, function and regulation. It also plays a role in humoral immunity. The BCMA gene is located at 16p13.1 and encodes a 184 amino acid type I transmembrane protein (Swiss prot ID Q02223), which contains a 54 amino acid extracellular domain, a 23 amino acid transmembrane domain, and a 107 amino acid extracellular domain.

TACI (Tumor necrosis factor receptor superfamily member 13B; Transmembrane activator and CAML interactor; CD267) is a transmembrane protein that is highly expressed in spleen, thymus, small intestine and peripheral blood leukocytes. It is also expressed by some B as well as T- cells and plays a role in their growth. Furthermore it is associated with the activation of a number of the transcription factors NFAT, API, and NF kappa B as well as being involved in humoral immunity. The TACI gene is located 17p11.2 and encodes a 293 amino acid.

APRIL and BAFF are TNF family proteins secreted by several cell types including those of the immune system but also epithelial cells, bone marrow osteoclasts and stroma cells. APRIL and BAFF are survival and proliferation factors, needed for correct B cell development and function. Moreover, expression of APRIL and BAFF is found in serum and tissue lesions of patients with chronic inflammatory diseases such as rheumatoid arthritis. Regarding stem cells, a recent study reported that BAFF and APRIL modulate the rate of adipogenic differentiation of hASC (Alexaki VI, Notas G, Pelekanou V et al. Adipocytes as immune cells: differential expression of TWEAK, BAFF, and APRIL and their receptors (Fn14, BAFF-R, TACI, and BCMA) at different stages of normal and pathological adipose tissue development. J Immunol 2009; 183:5948-5956.).

### BRIEF DESCRIPTION OF THE INVENTION

The invention provides new uses of culture media and methods for mesenchymal stem cells, which provide significant advantages over known uses of culture media and methods. The invention also provides related compositions. Additionally, the present document also discloses related culture media and culture medium supplements.

Thus, in a first aspect the invention relates to the use of a culture medium for expanding a population of mesenchymal stem cells, wherein the mesenchymal stem cells are derived from adipose tissue and wherein said medium comprises a BCMA & TACI ligand, wherein the BCMA & TACI ligand is selected from the group consisting of:
(i) a polypeptide that shows at least 85% identity to the polypeptide of SEQ ID NO:1,
(ii) a soluble polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a soluble polypeptide that shows at least 85% identity to the extracellular domain of the polypeptide of SEQ ID NO:2.

In a second aspect the invention relates to a composition comprising: (a) a culture medium; and (b) mesenchymal stem cells wherein the mesenchymal stem cells are derived from adipose tissue and wherein the culture medium comprises a BCMA & TACI ligand, wherein the BCMA & TACI ligand is selected from the group consisting of:
(i) a polypeptide that shows at least 85% identity to the polypeptide of SEQ ID NO:1,
(ii) a soluble polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a soluble polypeptide that shows at least 85% identity to the extracellular domain of the polypeptide of SEQ ID NO:2.

In a third aspect the invention relates to a method for expanding a population of mesenchymal stem cells wherein the mesenchymal stem cells are derived from adipose tissue, comprising: (a) providing a population of mesenchymal stem cells; (b) providing a culture medium; (c) contacting the mesenchymal stem cells with the culture medium; and (d) culturing the stem cells under appropriate conditions; said medium comprises a BCMA & TACI ligand, wherein the BCMA & TACI ligand is selected from the group consisting of:
(i) a polypeptide that shows at least 85% identity to the polypeptide of SEQ ID NO:1,
(ii) a soluble 5 polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a soluble polypeptide that shows at least 85% identity to the extracellular domain of the polypeptide of SEQ ID NO:2.

In a fourth aspect the invention relates to the use of a BCMA & TACI ligand in the manufacture of a mesenchymal stem cell therapy medicament, wherein the mesenchymal stem cells are derived from adipose tissue and wherein the BCMA & TACI ligand is selected from the group consisting of:
(i) a polypeptide that shows at least 85% identity to the polypeptide of SEQ ID NO:1,
(ii) a soluble 5 polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a soluble polypeptide that shows at least 85% identity to the extracellular domain of the polypeptide of SEQ ID NO:2.

In a fifth aspect the invention relates to a BCMA & TACI ligand for use as a mesenchymal stem cell therapy medicament, wherein the mesenchymal stem cells are derived from adipose tissue and wherein the BCMA & TACI ligand is selected from the group consisting of:
(i) a polypeptide that shows at least 85% identity to the polypeptide of SEQ ID NO:1,
(ii) a soluble 5 polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a soluble polypeptide that shows at least 85% identity to the extracellular domain of the polypeptide of SEQ ID NO:2.

Finally, in a sixth aspect the invention relates to the use of (a) a solid surface; and (b) a BCMA & TACI ligand, in a method for expanding a population of mesenchymal stem cells wherein the mesenchymal stem cells are derived from adipose tissue and wherein the BCMA & TACI ligand is selected from the group consisting of:
(i) a polypeptide that shows at least 85% identity to the polypeptide of SEQ ID NO:1,
(ii) a soluble 5 polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a soluble polypeptide that shows at least 85% identity to the extracellular domain of the polypeptide of SEQ ID NO:2.

### DEFINITIONS

In order to facilitate the understanding of the present description, the meaning of some terms and expressions in the context of the invention will be explained below. Further definitions will be included throughout the description as necessary

The term "allogeneic" as used herein shall be taken to mean from different individuals of the same species. Two or more individuals are said to be allogeneic to one another when the genes at one or more loci are not identical.

The term "autologous" as used herein shall be taken to mean from the same individual. The term 'population' of cells is any number of cells greater than 1 , but is preferably at least 1x10³ cells, at least 1x10⁴ cells, at least 1x10⁵ cells, at least 1x10⁶ cells, at least 1x10⁷ cells, at least 1x10⁸ cells, or at least 1x10⁹ cells. In preferred embodiments of the invention, at least 50%, at least 55%, at least 60%, at least 70%, at least 80%, at least 85%, at least 90%, at least 91%, at least 92%, at least 93%, at least 94% or at least 95%, of the stem cells (% by cell number) in an initial cell population will be undifferentiated multipotent stem cells.

The term "immune disease" refers to a condition in a subject characterized by cellular, tissue and/or organ injury caused by an immunological reaction of the subject. The term "autoimmune disease" refers to a condition in a subject characterized by cellular, tissue and/or organ injury caused by an immunological reaction of the subject to its own cells, tissues and/or organs. Illustrative, non-limiting examples of autoimmune diseases which can be treated with the immunomodulatory cells of the invention include alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CF1DS), chronic inflammatory demyelinating polyneuropathy, Churg-Strauss syndrome, cicatrical pemphigoid, CREST syndrome, cold agglutinin disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, Meniere's disease, mixed connective tissue disease, multiple sclerosis, type 1 or immune-mediated diabetes mellitus, myasthenia gravis, pemphigus vulgaris, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, sarcoidosis, scleroderma, progressive systemic sclerosis, Sjogren's syndrome, Good pasture's syndrome, stiff-man syndrome, systemic lupus erythematosus, lupus erythematosus, takayasu arteritis, temporal arteristis/giant cell arteritis, ulcerative colitis, uveitis, vasculitides such as dermatitis herpetiformis vasculitis, vitiligo, Wegener's granulomatosis, Anti-Glomerular Basement Membrane Disease, Antiphospholipid Syndrome, Autoimmune Diseases of the Nervous System , Familial Mediterranean Fever, Lambert-Eaton Myasthenic Syndrome, Sympathetic Ophthalmia, Polyendocrinopathies, Psoriasis, etc.

The term "Immune Mediated inflammatory Disease" shall be taken to mean any disease characterized by chronic or acute inflammation, resulting from, associated with or triggered by, a dysregulation of the normal immune response e.g. Crohn's disease, type 1 diabetes mellitus, rheumatoid arthritis, inflammatory bowel disease, psoriasis, psoriatic arthritis, ankylosing spondylitis, systemic lupus erythematosus, Hashimoto's disease, graft-versus-host disease, Sjogren's syndrome, pernicious anemia, Addison disease, scleroderma, Goodpasture's syndrome, ulcerative colitis, autoimmune hemolytic anemia, sterility, myasthenia gravis, multiple sclerosis, Basedow's disease, thrombopenia purpura, Guillain-Barré syndrome, allergy, asthma, atopic disease, arteriosclerosis, myocarditis, cardiomyopathy, glomerular nephritis, hypoplastic anemia, and rejection after organ transplantation.

For the purposes of the invention described herein, "immune disorders" include autoimmune diseases and immunologically mediated diseases.

The term "inflammatory disease" refers to a condition in a subject characterized by inflammation, e.g., chronic inflammation. Illustrative, non-limiting examples of inflammatory disorders include, but are not limited to, Celiac Disease, rheumatoid arthritis (RA), Inflammatory Bowel Disease (IBD), asthma, encephalitis, chronic obstructive pulmonary disease (COPD), inflammatory osteolysis, allergic disorders, septic shock, pulmonary fibrosis (e.g. , idiopathic pulmonary fibrosis), inflammatory vacultides (e.g. , polyarteritis nodosa, Wegner's granulomatosis, Takayasu's arteritis, temporal arteritis, and lymphomatoid granulomatosus), post-traumatic vascular angioplasty (e.g. , restenosis after angioplasty), undifferentiated spondyloarthropathy, undifferentiated arthropathy, arthritis, inflammatory osteolysis, chronic hepatitis, and chronic inflammation resulting from chronic viral or bacteria infections.
The term "isolated" applied to a cell population refers to a cell population, isolated from the human or animal body, which is substantially free of one or more cell populations that are associated with said cell population in vivo or in vitro.

The term "MHC" (major histocompatibility complex) refers to a subset of genes that encodes cell-surface antigen-presenting proteins. In humans, these genes are referred to as human leukocyte antigen (HLA) genes. Herein, the abbreviations MHC or HLA are used interchangeably. The term "subject" refers to an animal, preferably a mammal including a non-primate (e.g., a cow, pig, horse, cat, dog, rat, or mouse) and a primate (e.g., a monkey, or a human). In a preferred embodiment, the subject is a human.

The term "immunomodulatory" refers to the inhibition or reduction of one or more biological activities of the immune system which includes, but is not limited to, downregulation of immune response and inflammatory states as well as changes in cytokine profile, cytotoxic activity and antibody production. The term "antigen specific immunomodulatory" refers to the inhibition or reduction of one or more biological activities of the immune system associated with a specific antigen or antigens, including both alloantigens and autoantigens.

As used herein, "negative" or "-" as used with respect to cell surface markers shall be taken to mean that, in a cell population, less than 20%, 10%, preferably less than 9%, 8%, 7%, 6%, 5%, 4%, 3%, 2%, 1 % or none of the cells express said marker. Expression of cell surface markers may be determined for example by means of flow cytometry for a specific cell surface marker using conventional methods and apparatus (for example a Beckman Coulter Epics XL FACS system used with commercially available antibodies and standard protocols known in the art).

As used herein the term "stem cell" shall be taken to mean a cell which is capable of giving rise to multiple different types of cells.

The term "mesenchymal stem cell" or "MSC" shall be taken to mean a stem cell originally derived from the mesenchyme. The term refers to a cell which is capable of differentiating into at least two or more of an osteoblast, a chondrocyte, an adipocyte, or a myocyte. MSCs may be isolated from any type of tissue. Generally MSCs will be isolated from bone marrow, adipose tissue, umbilical cord, or peripheral blood. The MSCs used in the invention may in some embodiments be isolated from bone marrow (BM-MSC) or adipose tissue (ASC). In a preferred aspect of the invention, MSCs are obtained from lipoaspirates, themselves obtained from adipose tissue.

As used herein the terms "multipotent" (alternatively known as "pluripotent") shall be taken to mean a cell which is capable of giving rise to multiple types of cells of different lineages.

As used herein, the expression "significant expression" or its equivalent terms "positive" and "+" when used in regard to a cell surface marker shall be taken to mean that, in a cell population, more than 20%, preferably more than, 30%, 40%, 50%, 60%, 70%, 80%, 90% 95%, 98%, 99% or even all of the cells of the cells express said marker.

Expression of cell surface markers may be determined for example by means of flow cytometry for a specific cell surface marker using conventional methods and apparatus (for example a Beckman Coulter Epics XL FACS system used with commercially available antibodies and standard protocols known in the art) that show a signal for a specific cell surface marker in flow cytometry above the background signal using conventional methods and apparatus (for example, a Beckman Coulter Epics XL FACS system used with commercially available antibodies and standard protocols known in the art). The background signal is defined as the signal intensity given by a non-specific antibody of the same isotype as the specific antibody used to detect each surface marker in conventional FACS analysis. For a marker to be considered positive the specific signal observed is stronger than 20%, preferably stronger than, 30%, 40%, 50%, 60%, 70%, 80%, 90%, 500%, 1000%, 5000%, 10000% or above, than the background signal intensity using conventional methods and apparatus (for example a Beckman Coulter Epics XL FACS system used with commercially available antibodies and standard protocols known in the art).

Furthermore, commercially available and known monoclonal antibodies against said cell-surface markers (e.g., cellular receptors and transmembrane proteins) can be used to identify relevant cells.

As used herein, the terms "treat", "treatment" and "treating" when used directly in reference to a patient or subject shall be taken to mean the amelioration of one or more symptoms associated with a disorder including, but not limited to, an inflammatory disorder, an autoimmune disease or an immunologically mediated disease including rejection of transplanted organs and tissues, wherein said amelioration results from the administration of the immunomodulatory cells of the invention, or a pharmaceutical composition comprising same, to a subject in need of said treatment.

As used herein the terms "repair" and "repairing" when used directly in reference to damaged tissues shall be taken to mean the amelioration of such damage by both direct mechanisms such as the regeneration of damaged tissues, as well as through indirect mechanisms e.g., reducing inflammation thereby enabling tissue formation.

As used herein the term "inducer" when used in reference to a ligand shall be taken to mean an agent or agents that results in the increased production of said ligand.

As used herein the term "cellular therapy" shall be taken to mean the transplantation of human or animal cells to prevent, treat or ameliorating one or more symptoms associated with a disease or disorder, such as but not limited to the replacement or repair of damaged tissues or organs, the modulation of immune reactions and the reduction of inflammatory symptoms.

As used herein the term BCMA is taken to mean a polypeptide that comprises a sequence at least 85% identical (preferably, at least 90%, 95%, 98%, 99%, or 100% identical) to the amino acid sequence according to SEQ ID NO: 3.

As used herein the term TACI is taken to mean a polypeptide that comprises a sequence at least 85% identical (preferably, at least 90%, 95%, 98%, 99%, or 100% identical) to the amino acid sequence according to SEQ ID NO: 4.

The present invention discloses that the use of a BCMA and TACI ligand and/or inducer thereof improves multipotent stem cell culture. Accordingly, the culture media of the invention may comprise a BCMA and TACI ligand and/or inducer thereof, and the invention provides the use of a BCMA and TACI ligand and/or inducer thereof for multipotent stem cell culture.

A 'BCMA & TACI ligand' is an agent that binds to at least one BCMA isoform and at least one TACI isoform. Various methods for determining if a given substance is a ligand are known and might be used in conjunction with this invention.

Particularly preferred is a BCMA & TACI agonist. A 'BCMA & TACI agonist' is an agent that binds to at least one BCMA isoform and at least one TACI isoform and trigger a physiological response.

Particularly preferred BCMA & TACI ligands according to the present invention are BAFF and APRIL.

As used herein, "BAFF" is a polypeptide that comprises a sequence at least 85% identical (preferably, at least 90%, 95%, 98%, 99%, or 100% identical) to the BAFF amino acid. In preferred embodiments, the BAFF is a soluble polypeptide comprising all or a substantial part (e.g., at least 85%, 90%, 95% or greater) of the TNF-like domain of BAFF.

The amino acid and nucleic acid sequences of naturally occurring full-length human BAFF are available under GenBank™ accession No. Q9Y275 (SEQ ID NO:1). Full-length BAFF is a type II membrane protein having intracellular, transmembrane, and extracellular domains. In human BAFF, these domains are comprised approximately (e.g., ±2 or 3 residues) of amino acids 1-46, 47-67, and 68-285 of SEQ ID NO:1, respectively. A naturally occurring soluble form of BAFF exists, in which proteolytic cleavage occurs between amino acids R133 and A134 in human BAFF, resulting in a water soluble biologically active C-terminal portion of BAFF.

Compositions suitable for use in methods of the invention include soluble BAFF. Such soluble forms of BAFF generally do not comprise the transmembrane and intracellular domains. Since naturally occurring soluble BAFF does not comprise a portion of the extracellular domain (i.e., amino acids 74-133 of SEQ ID NO:1) soluble BAFF of the invention may likewise exclude these regions.

In certain embodiments, the soluble BAFF is a polypeptide comprising all or a substantial part of the TNF- like domain of BAFF, e.g., amino acids 145-284 of SEQ ID NO:1 (human BAFF), or a sequence at least 85%, 90%, or 95% identical thereto.

In non-limiting illustrative embodiments, soluble BAFF comprises amino acids 134-285 of SEQ ID NO:1, or N- and/or C-terminal truncations thereof. For example, the N-terminus of soluble BAFF may be between residues 134-170 of SEQ ID NO:1, e.g., the N-terminus of soluble BAFF may extend up to and include amino acid 134, 135, 136, 137, 138, 139, 140, 141 , 142, 143, 144, 145, 146, 147, 148, 149, 150, 151 , 152, 153, 154, 155, 156, 157, 158, 159, 160, 161 , 162, 163, 164, 165, 166, 167, 168, 169, or 170; while independently, the C- terminus be between residues 250-285 of SEQ ID NO:1, e.g., it may extend up to and include amino acid 285, 284, 283, 282, 281 , 280, 279, 278, 277, 276, 275, 274, 273, 272, 271 , 270, 269, 268, 267, 266, 265, 264, 263, 262, 261 , 260, 259, 258, 257, 256, 255, 254, 253, 252, 251 , 250 of SEQ ID NO:1. In one embodiment, soluble BAFF comprises amino acids 136-285 of SEQ ID NO:1.

A particularly preferred inducer of BAFF according to the present invention is interferon gamma.

As used herein, "APRIL" is a polypeptide that comprises a sequence at least 85% identical (preferably, at least 90%, 95%, 98%, 99%, or 100% identical) to the extracellular domain of APRIL. In certain embodiments, APRIL is a polypeptide comprising all or a substantial part (e.g., at least 85%, 90%, 95% or greater) of the extracellular domain of APRIL.

The amino acid and nucleic acid sequences of naturally occurring full-length human APRIL are available under GenBank™ accession No. 075888 (SEQ ID NO:2). Full-length APRIL is a type II membrane protein having intracellular, transmembrane, and extracellular domains. In human APRIL these domains are comprised approximately (e.g., ±2 or 3 residues) of amino acids 1-28, 29-49, and 50-250 of SEQ ID NO:2 , respectively. A naturally occurring soluble form of APRIL exists, in which cleavage occurs between amino acids R104 and A105 in human APRIL, resulting in a soluble biologically active C-terminal portion of APRIL.

Compositions suitable for use in methods of the invention include soluble APRIL. Such soluble forms of APRIL generally do not comprise the transmembrane domain. Since naturally occurring soluble APRIL does not comprise a portion of the extracellular domain (i.e., amino acids 49-104 of SEQ ID NO:2), soluble APRIL of the invention may likewise exclude these regions.

In non-limiting illustrative embodiments, soluble APRIL comprises amino acids 105-250 of SEQ ID NO:2, or N- and/or C-terminal truncations thereof. For example, the N-terminus of soluble APRIL may be between residues 105-140 of SEQ ID NO:2, e.g., the N-terminus of soluble APRIL may extend up to and include amino acid105, 106, 107, 108, 109, 110, 112, 113 114, 115, 116, 117, 118, 119, 120, 121, 122, 123, 124, 125, 126, 127, 128, 129, 130, 131, 132, 133, 134, 135, 136, 137, 138, 139 or 140 ; while independently, the C- terminus be between residues 220-250 of SEQ ID NO:2, e.g., it may extend up to and include amino acid 285, 284, 225, 226, 227, 228, 229, 230, 231, 232, 233, 234, 235, 236, 237, 238, 239, 240, 241, 242, 243, 244, 245, 246, 247, 248, 249, or 250 of SEQ ID NO:2. In one embodiment, soluble APRIL comprises amino acids 107-250 of SEQ ID NO:2.

A particularly preferred inducer of BAFF according to the present invention is CXCL-12.

Composition comprising BAFF or APRIL suitable for use in the methods of the invention further include such derivatives thereof (also referred to herein as "substitutes") in which the amino acid sequence is mutated, partially deleted, and/or contains one or more insertions so long as changes to the wild type sequence do not substantially affect the biological activity of the molecule with respect to the BCMA and TACI receptors.

The invention provides new uses of culture media and methods for multipotent stem cells, which provide significant advantages over known uses of culture media and methods. The invention also provides related compositions. Additionally, the present document also discloses related culture media and culture medium supplements.

### BRIEF DESCRIPTION OF THE FIGURES

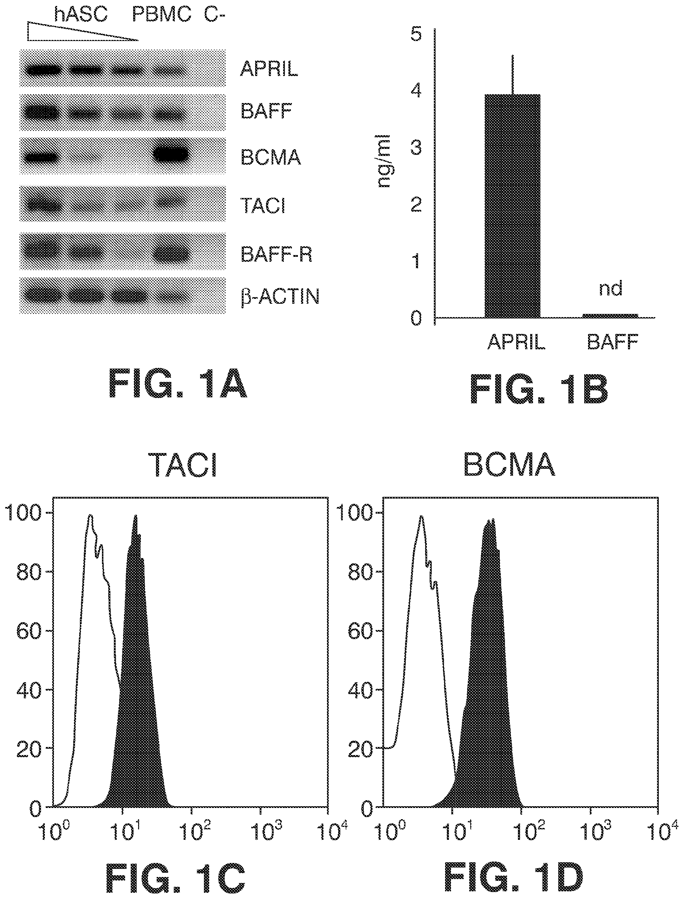

**Figure 1****. Expression of APRIL, BAFF, and their receptors in hASC.** (A) RNA was extracted and reverse-transcribed from cultured hASC. Peripheral blood mononuclear cells (PBMC) were purified from buffy coats and used as positive control. The figure shows the amplified PCR product specific for each gene (APRIL, BAFF, TACI, BCMA, BAFF-R) with β-actin as control and cDNA serial dilutions (1, 1:5, 1:25). Three independent assays were performed per target and experiment. (B) APRIL and BAFF concentration in hASC supernatants was quantified by ELISA (n = 6, day 6). Mean ± SD shown for three independent experiments. (C) TACI and BCMA receptor expression was determined by flow cytometry. Filled histograms represent hASC stained with TACI and BCMA fluorochrome-coupled antibodies (isotype controls, empty histograms). Data were anayzed using the FlowJo program (TreeStar, Ashland, OR).

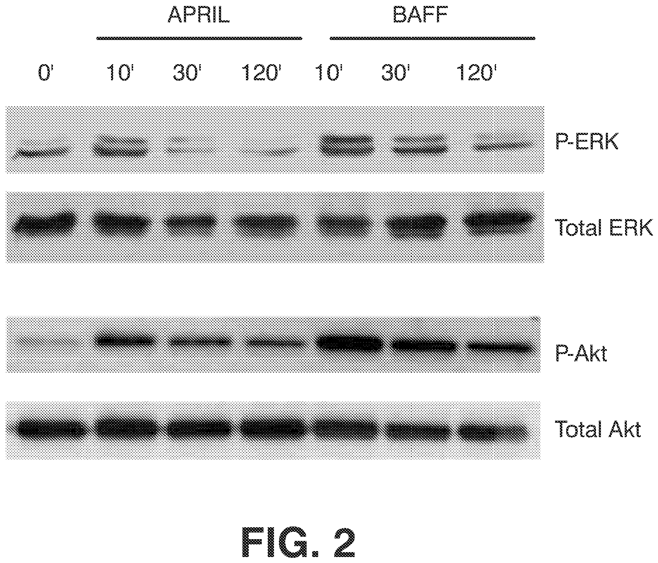

**Figure 2****. APRIL and BAFF enhance hASC viability and proliferation.** (A): Cell viability was assessed by the CellTiter AQueous One Solution Cell Proliferation Assay (Promega, Madison, WI) according to manufacturer's protocols. hASC (3.5 x 103 cells/well) were cultured for 6 days in 96-well plates with 100 ng/ml APRIL or BAFF. Absorbance (OD 490 nm) was measured in an ELISA plate reader (n = at least three experiments). Values were normalized to the absorbance of cells cultured without stimuli (100%). (B): Histograms show cell proliferation measured by a colorimetric BrdU incorporation assay. hASC were cultured in p60 plates with 100 ng/ml APRIL or BAFF; BrdU (20 µM) was added for the last three days. At day 6, BrdU incorporation was detected by flow cytometry using an anti-BrdU antibody. Numerals indicate percentage of cells positive for BrdU and, therefore, actively proliferating.

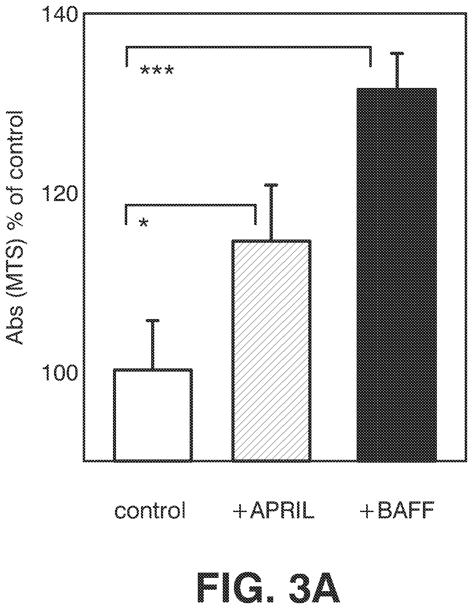

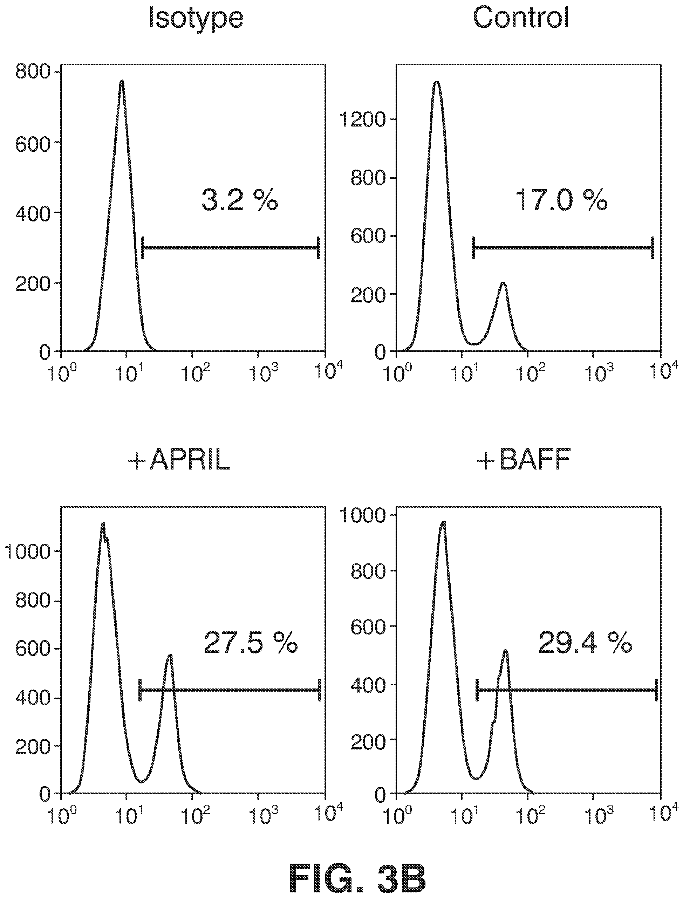

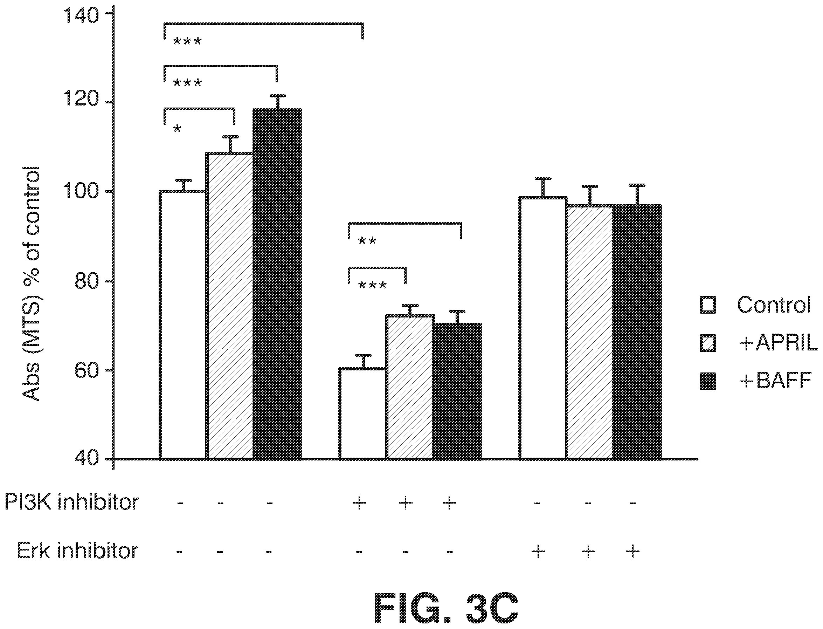

**Figure 3****. APRIL and BAFF-mediated signaling in hASC.** hASC were stimulated with 100 ng/ml APRIL or BAFF (10, 30, 60, 120 min, 37°C), placed on ice, washed with ice-cold PBS, and lysed with cell lysis buffer (Roche, Indianapolis, IN). Extracts were quantified by absorbance, resolved in SDS-PAGE and analyzed by immunoblot. Antibodies used were anti-phospho-Akt (Ser473), -Akt, -phospho-ERK1/2 (Thr202/Tyr204), and -ERK1/2) (BD Bioscience); secondary antibodies were horseradish peroxidase-conjugated bovine anti-rabbit and goat anti-mouse IgG. (B): Histograms show cell proliferation measured by BrdU incorporation as in (Fig. 2B), with addition of the ERK inhibitor U0126 (2.5 µM; Sigma) or the PI3K inhibitor LY294002 (10 µM; Calbiochem) to the culture on days 0 and 3. Results represent one of three independent experiments

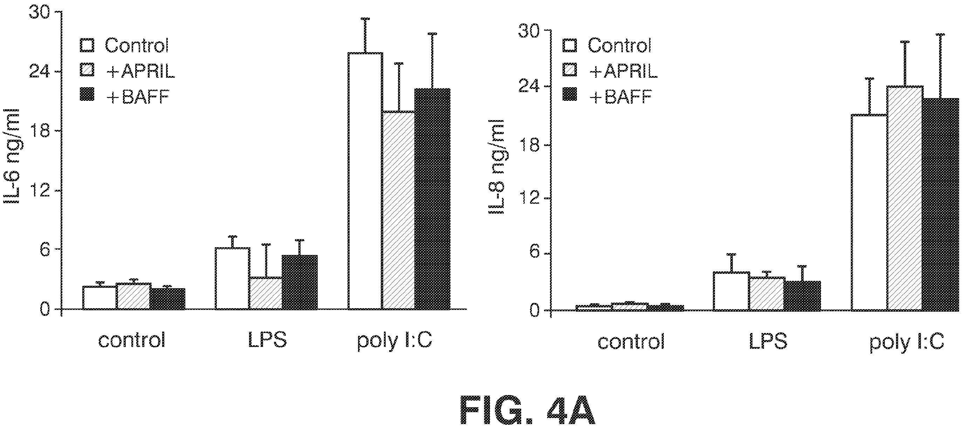

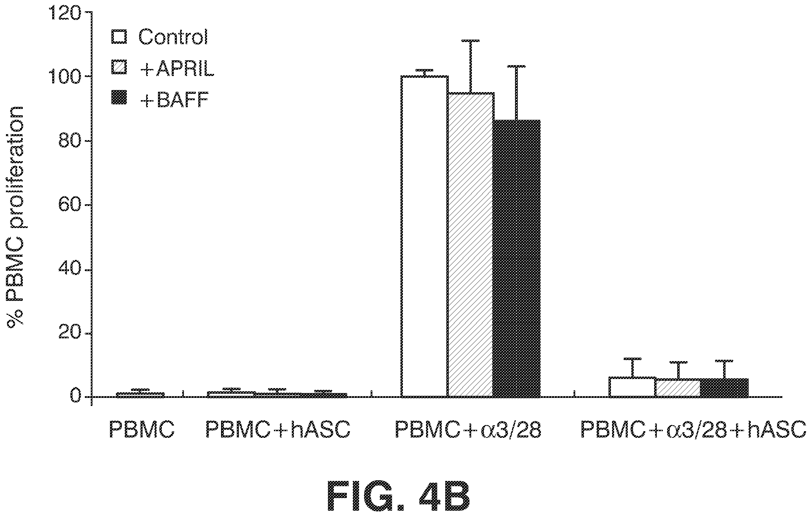

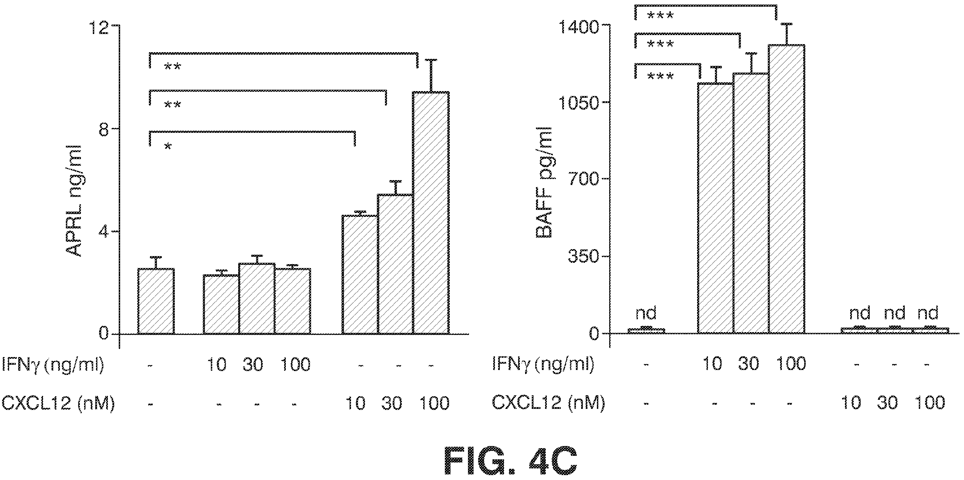

**Figure 4****. Modulation of APRIL and BAFF in hASC.** Immunosupresive effect (A): hASC cells were cultured with 100 ng/ml APRIL (light grey bar), BAFF (dark grey bar) or no stimulus (white bar) and then stimulated with 1 µg/ml LPS or poly I:C. The figure shows IL-6 and IL-8 concentration in the medium after 48 h, measured by the Cytometric Bead Array (CBA) immunoassay (BD Bioscience) following manufacturer instructions. Data were acquired using a FACScalibur cytometer and analyzed using FCAP array software (BD Bioscience). (B): Immunosupression assay: Allogeneic hASCs were cultured with activated CFSE labelled PBLs at a ratio of 1:25 (hASC: PBLs) in the presence or absence of BAFF or APRIL (100ng/ml). After 5 days cells were harvested, analyzed by flow cytometry in a FACScalibur cytometer (BD Bioscience) and acquired for CFSE. Percentage of proliferating cells (relative to PBLs alone) in the last two generations is shown. Mean and standard deviation of experiments with 6 different buffy coats are represented. Buffy coats were provided by the National Transfusion Centre of the Comunidad Autónoma of Madrid. PBLs were isolated by density centrifugation gradient using Ficollplaque Plus (GE Healthcare Biosciences AB, Uppsala) (C): hASC cells were cultured with IFNγ. Supernatants were collected five days later. The figure shows ELISA measurement of APRIL and BAFF concentrations in the medium (R&D Systems for BAFF, Bender MedSystem for APRIL).

### DETAILED DESCRIPTON OF THE INVENTION

The present invention can be used to provide ex-vivo expanded populations of stem cells, which can be used for applications in cellular therapy. There is a long-felt need in the art for an efficient expansion method to provide the large number of stem cells required for cell therapy. An advantage of the various embodiments of the invention is that they can be used to culture mesenchymal stem cells at a high proliferation rate, whilst maintaining their undifferentiated phenotype.

The culture media disclosed herein comprise, amongst other ingredients, a BCMA and TACI ligand and/or inducer thereof.

Accordingly, the disclosure provides a culture medium for expanding a population of multipotent stem cells, which comprises a BCMA and TACI ligand and/or inducer thereof.

The disclosure also provides a culture medium supplement that comprises a BCMA and TACI ligand and/or inducer thereof.

The disclosure also provides a hermetically-sealed vessel containing a culture medium or culture medium supplement of the invention.

The disclosure also provides a method for preparing a culture medium as disclosed herein, comprising the steps of: (a) obtaining a culture medium; and (b) adding a BCMA and TACI ligand and/or inducer thereof to the culture medium.

In one aspect, the invention provides the use of a culture medium of the disclosure for expanding a population of mesenchymal stem cells.

The invention also provides a composition comprising: (a) a culture medium according to the disclosure; and (b) stem cells.

The invention also provides a composition containing: (a) a culture medium according to the disclosure; and (b) a solid surface.

The invention also provides an ex-vivo method for expanding a population of multipotent stem cells, comprising: (a) providing a population of multipotent stem cells; (b) providing a culture medium of the invention; (c) contacting the stem cells with the culture medium; and (d) culturing the cells under appropriate conditions.

In one aspect the invention provides the use of a BCMA & TACI ligand and/or inducer thereof in the manufacture of a cellular therapy medicament.

Accordingly in one embodiment the invention also provides a method of manufacture of a cellular therapy medicament, comprising: (a) providing a population of multipotent stem cells; (b) providing a culture medium of the invention; (c) contacting the stem cells with the culture medium; and (d) culturing the cells under appropriate conditions.

The invention also provides the use of a composition comprising: (a) a culture medium according to the invention; and (b) stem cells, for manufacturing a cellular therapy medicament.

The invention also provides the use of a composition comprising: (a) a culture medium according to the invention; and (b) a solid surface, for manufacturing a cellular therapy medicament.

Said medicaments are of use in the treatment, repair, prophylaxis, and/or amelioration of damaged tissues, or one or more symptoms associated with inflammatory and/or immune disorders such as but not limited to autoimmune diseases, inflammatory disorders, and immunologically mediated diseases including rejection of transplanted organs and tissues. A cellular therapy medicament of the invention comprises a prophylactically or therapeutically effective amount of stem cells and a pharmaceutical carrier. Particularly preferred are stem cells of mesenchymal origin, most preferably adipose-derived stem cells.

Examples of dosages and dosage regimens for each of these cell types are known in the art. Suitable pharmaceutical carriers are known in the art and are preferably those approved by a regulatory agency of the US Federal or a state government or listed in the U S Pharmacopeia, or European Pharmacopeia, or other generally recognized pharmacopeia for use in animals, and more particularly in humans. The term "carrier" refers to a diluent, adjuvant, excipient, or vehicle with which the therapeutic agent is administered. The composition, if desired, can also contain minor amounts of pH buffering agents. Examples of suitable pharmaceutical carriers are described in "Remington's Pharmaceutical Sciences" by E W Martin. Such compositions will contain a prophylactically or therapeutically effective amount of a prophylactic or therapeutic agent preferably in purified form, together with a suitable amount of carrier so as to provide the form for proper administration to the subject. The formulation should suit the mode of administration. In a preferred embodiment, the medicaments are sterile and in suitable form for administration to a subject, preferably an animal subject, more preferably a mammalian subject, and most preferably a human subject.

The medicament of the invention may be in a variety of forms. These include, for example, semi-solid, and liquid dosage forms, such as lyophilized preparations, liquid solutions or suspensions, injectable and infusible solutions, etc., the medicament is preferably injectable.

It is preferred that the medicaments are for treating or repairing damaged tissue (preferably mesenchymal tissue), and/or for the treatment, modulation, prophylaxis, and/or amelioration of one or more symptoms associated with inflammatory and/or immune disorders. Accordingly the methods and cells of the invention are of use in the treatment of any disorder characterized by either or all of said symptoms. A representative non-exhaustive list of such disorders is provided in the definitions section. Particularly preferred is a medicament for the treatment of immune-mediated inflammatory diseases. Further preferred is a medicament for the treatment of diabetes mellitus, rheumatoid arthritis (RA), inflammatory bowel disease (IBD, including Crohn's disease and/or Ulcerative Colitis) and multiple sclerosis (MS).

The invention also provides the use a BCMA and TACI ligand and/or inducer thereof for multipotent stem cell culture.

The specific ingredients of the culture media, supplements and compositions of the invention can vary according to particular needs and applications. Likewise, the precise steps of the methods of the invention can vary according to particular needs and applications. The culture media, supplements, methods, compositions and uses according to this invention may be optimised by routine experimentation. For example, if a culture medium, supplement or composition fails to give the desired level of multipotent stem cell expansion, variables such as the amount of each ingredient in the culture medium or supplement, seeding densities, culture conditions, culture periods, etc. can be altered in further experiments. The amount of each of the ingredients described herein can be optimised independently of the other ingredients by routine optimisation or one or more ingredients can be added or removed. A culture medium can be tested for its ability to support expansion of multipotent stem cells by testing it alongside or in place of a known culture medium or method.

The culture media, supplements, methods, compositions and uses of the invention are described in more detail below. The practice of the present invention will employ, unless otherwise indicated, conventional techniques of cell culture, molecular biology and microbiology, which are within the skill of those working in the art.

Numerous textbooks are available that provide guidance on mammalian cell culture media and methods, including textbooks dedicated to culture media and methods for stem cells.

### Culture Media

The culture media for use in the invention comprises a BCMA & TACI ligand and/or inducer thereof. In one aspect the culture media of the invention comprises BAFF or a BAFF substitute. In an alternative aspect the culture media of the invention comprises APRIL or an APRIL substitute. In a further aspect the culture media of the invention comprises APRIL and BAFF substitutes thereof.

The culture media for use in the invention may comprise two or more, three or more, four or more, five or more, six or more, seven or more, eight or more, nine or more, ten or more, eleven or more, or twelve or more, different BCMA & TACI ligands and/or inducer thereof.

A culture medium for use in the invention may comprise between about lOpM and about 100 mM of a BCMA & TACI ligand and/or inducer thereof.

In some embodiments, a culture medium for use in the invention may comprise between about 100nM and about 1 mM of a BCMA & TACI ligand and/or inducer thereof. For example, a culture medium may comprise between about 1pg/ml and 1-10µg/ml. of a BCMA & TACI ligand and/or inducer thereof, between about 10 and about 700 ng/ml of a BCMA & TACI ligand and/or inducer thereof, between about 50 and about 65Oµg/ml of a BCMA & TACI ligand and/or inducer thereof, between about 50 and about 40Oµg/ml of a BCMA & TACI ligand and/or inducer thereof, between about 50 and about 200µg/ml of a BCMA & TACI ligand and/or inducer thereof, or about 100 µg/ml of a BCMA & TACI ligand and/or inducer thereof. For example, a culture medium may comprise between about 50 and about 15Oµg/ml.

Cell culture media typically contain a large number of ingredients, which are necessary to support maintenance of the cultured cells. A culture medium of the invention will therefore normally contain many other ingredients in addition to a BCMA & TACI ligand and/or inducer thereof. Suitable combinations of ingredients can readily be formulated by the skilled person, taking into account the following disclosure. A culture medium according to the invention will generally be a nutrient solution comprising standard cell culture ingredients, such as amino acids, vitamins, inorganic salts, a carbon energy source, and a buffer, as described in more detail below.

A culture medium according for use in the invention may be generated by modification of an existing cell culture medium. The skilled person understands the types of culture media that might be used for multipotent stem cell culture. Potentially suitable cell culture media are available commercially, and include Dulbecco's Modified Eagle Media (DMEM), Minimal Essential Medium (MEM), Minimal Essential Medium Eagle, Knockout-DMEM (KO-DMEM), Glasgow Minimal Essential Medium (G-MEM), Basal Medium Eagle (BME), DMEM/Ham's F 12, Advanced DMEM/Ham's F 12, Iscove's Modified Dulbecco's Media and Minimal Essential Media (MEM).

Many cell culture media already contain amino acids, however some require supplementation prior to culturing of cells. Amino acids which may be present include L-alanine, L- arginine, L-asparagine, L-aspartic acid, L-cysteine, L-cystine, L-glutamic acid, L- glutamine, glycine, L-histidine, L-isoleucine, L-leucine, L-lysine, L-methionine, L- phenylalanine, L-proline, L-serine, L-threonine, L-tryptophan, L-tyrosine, L-valine and combinations thereof.

Generally, each amino acid when present is present at about 0.001 to about 1 g/L of medium (usually at about 0.01 to about 0.15 g/L), except for L-glutamine which is present at about 0.05 to about 1 g/L (usually about 0.1 to about 0.75 g/L). The amino acids may be of synthetic origin.

A culture medium for use in the invention may comprise one or more vitamins. The skilled person understands the appropriate types and amounts of vitamins for use in stem cell culture media. Vitamins which may be present include thiamine (vitamin B1), riboflavin (vitamin B2), niacin (vitamin B3), D-calcium pantothenate (vitamin B5), pyridoxal/pyridoxamine/pyridoxine (vitamin B6), folic acid (vitamin B9), cyanocobalamin (vitamin B 12), ascorbic acid (vitamin C), calciferol (vitamin D2), DL-alpha tocopherol (vitamin E), biotin (vitamin H) and menadione (vitamin K).

A culture medium for use in the invention may comprise one or more inorganic salts. The skilled person understands the appropriate types and amounts of inorganic salts for use in stem cell culture media. Inorganic salts are typically included in culture media to aid maintenance of the osmotic balance of the cells and to help regulate membrane potential. Inorganic salts which may be present include salts of calcium, copper, iron, magnesium, potassium, sodium, zinc. The salts are normally used in the form of chlorides, phosphates, sulphates, nitrates and bicarbonates. Specific salts that may be used include CaCl₂, CuSO₄-5H₂O, Fe(NO₃)-9H₂O, FeSO₄-7H₂O, MgCl, MgSO₄, KCl, NaHCO₃, NaCl, Na₂HPO₄, Na₂HPO₄-H₂O and ZnSO₄-7H₂O.

The osmolarity of the medium may be in the range from about 200 to about 400 mθsm/kg, in the range from about 290 to about 350 mθsm/kg, or in the range from about 280 to about 310 mθsm/kg. The osmolarity of the medium may be less than about 300 mθsm/kg (e.g. about 280 mθsm/kg).

A culture medium for use in the invention may comprise a carbon energy source, in the form of one or more sugars. The skilled person understands the appropriate types and amounts of sugars to use in stem cell culture media. Sugars which may be present include glucose, galactose, maltose and fructose. The sugar is preferably glucose, particularly D-glucose (dextrose). A carbon energy source will normally be present at between about 1 and about 10 g/L.

A culture medium for use in the invention may comprise a buffer. A suitable buffer can readily be selected by the skilled person. The buffer may be capable of maintaining the pH of the culture medium in the range about 6.5 to about 7.5 during normal culturing conditions, most preferably around pH 7.0. Buffers that may be used include carbonates (e.g. NaHCO₃), chlorides (e.g. CaCl₂), sulphates (e.g. MgSO₄) and phosphates (e.g. NaH₂PO₄). These buffers are generally used at about 50 to about 500 mg/l. Other buffers such as N-[2-hydroxyethyl]-piperazine-N'-[2-ethanesul-phonic acid] (HEPES) and 3-[N- morpholinoj-propanesulfonic acid (MOPS) may also be used, normally at around 1000 to around 10,000 mg/l.

A culture medium for use in the invention may contain serum. Serum contains cellular and non-cellular factors and components that may be necessary for viability and expansion. Serum obtained from any appropriate source may be used, including fetal bovine serum (FBS), bovine serum (BS), calf serum (CS), fetal calf serum (FCS), newborn calf serum (NCS), goat serum (GS), horse serum (HS), porcine serum, sheep serum, rabbit serum, rat serum (RS), etc. It is also within the scope of the invention that if said MSC are of human origin, the cell culture medium is supplemented with a human serum, preferably of autologous origin. It is understood that sera can be heat inactivated at 55-65deg. C if deemed necessary to inactivate components of the complement cascade. Where a serum replacement is used, it may be used at between about 2% and about 25% by volume of the medium, according to conventional techniques.

In other disclosures, a culture medium for use in the invention may contain a serum replacement. Various different serum replacement formulations are commercially available and are known to the skilled person, such as but not limited to serum albumin, serum transferrin, selenium, and recombinant proteins including but not limited to insulin, platelet-derived growth factor (PDGF), and basic fibroblast growth factor (bFGF).Where a serum replacement is used, it may be used at between about 2% and about 25% by volume of the medium, according to conventional techniques.

In other disclosures, a culture medium for use in the invention may be serum-free and/or serum replacement-free. A serum-free medium is one that contains no animal serum of any type. Serum-free media may be preferred to avoid possible xeno-contamination of the stem cells. A serum replacement-free medium is one that has not been supplemented with any commercial serum replacement formulation.

A culture medium for use in the invention may comprise one or more hormones and include, but are not limited to, D-aldosterone, diethylstilbestrol (DES), dexamethasone, b-estradiol, hydrocortisone, insulin, prolactin, progesterone, somatostatin/human growth hormone (HGH), etc.

A culture medium for use in the invention may comprise one or more cytokines. The skilled person understands the appropriate types and amounts of cytokines for use in stem cell culture media. Cytokines which may be present include both early acting and late acting cytokines and may be selected from the group consisting of stem cell factor, FLT3 ligand, interleukin-6, thrombopoietin, interleukin-3, granulocyte colony stimulating factor, granulocyte/macrophage colony stimulating factor and erythropoietin.

A culture medium may further comprise phenol red as a pH indicator, to enable the status of the medium to be easily monitored (e.g. at about 5 to about 50 mg/litre).

Preferred culture media are described in the Examples herein. In one disclosure, a culture medium of the invention comprises for example, Dulbecco's Modified Eagle's Medium (DMEM), with antibiotics (for example, 100units/ml Penicillin and 100[mu]g/ml Streptomycin) or without antibiotics, and 2 mM glutamine, and supplemented with 2-20% fetal bovine serum (FBS), and 100 µg/ml BAFF and/or APRIL.

A culture medium of the disclosure will normally be formulated in deionized, distilled water. A culture medium of the invention will typically be sterilized prior to use to prevent contamination, e.g. by ultraviolet light, heating, irradiation or filtration. The culture medium may be frozen (e.g. at -20°C or -80°C) for storage or transport.

Antimicrobial agents are also typically used in cell culture to mitigate bacterial, mycoplasmal, and fungal contamination. The medium may contain one or more antimicrobial agents or antibiotics to prevent contamination. Typically, antibiotics or anti-mycotic compounds used are mixtures of penicillin/streptomycin, but can also include, but are not limited to amphotericin (Fungizone(R)), ampicilhn, gentamicin, bleomycin, hygromacin, kanamycin, mitomycin, etc.

The medium may have an endotoxin content of less than 0.1 endotoxin units per ml, or may have an endotoxin content less than 0.05 endotoxin units per ml. Methods for determining the endotoxin content of culture media are known in the art.

The culture medium may be conditioned by the addition of cells constitutively expressing and secreting a a BCMA or TACI ligand such as BAFF or APRIL or substitutes thereof. Conditioned medium is produced by culturing a population of said cells in a culture medium for a time sufficient to condition the medium, then harvesting the conditioned medium. Where a conditioned medium is used, the medium may be conditioned on mammalian cells, e.g. mouse cells or human cells. Various different types of mammalian cells may be used to produce conditioned medium suitable for multipotent stem cell culture.

A culture medium may be a 1x formulation or a concentrated formulation, e.g. a 2x to 250x concentrated medium formulation. In a Ix formulation each ingredient in the medium is at the concentration intended for cell culture. In a concentrated formulation one or more of the ingredients is present at a higher concentration than intended for cell culture. Concentrated culture media is well known in the art. Culture media can be concentrated using known methods e.g. salt precipitation or selective filtration. A concentrated medium may be diluted for use with water (preferably deionized and distilled) or any appropriate solution, e.g. an aqueous saline solution, an aqueous buffer or a culture medium.

A culture medium as disclosed herein may be capable of expanding a population of stem cells in a multipotent, undifferentiated and proliferative state for at least 2 passages under appropriate conditions. Stem cells are considered to be in a multipotent, undifferentiated and proliferative state if they exhibit certain characteristics as described in more detail elsewhere herein. Appropriate conditions can be selected by the skilled person from those normally used for multipotent stem cell culture. Preferably, a culture medium is capable of expanding a population of stem cells in a multipotent, undifferentiated and proliferative state for at least 3, at least 4, at least 5, at least 6, at least 7, at least 8, at least 9, at least 10, at least 20, at least 30, at least 40, at least 50, at least 60, at least 70, at least 80, at least 90 or at least 100, passages under appropriate conditions. A culture medium may be capable of expanding a population of multipotent stem cells in a multipotent, undifferentiated and proliferative state for more than 3 passages, more than 4 passages, more than 5 passages, more than 10 passages, more than 15 passages, more than 20 passages, more than 25 passages, more than 30 passages, more than 40 passages, more than 50 passages, or more than 100 passages. Accordingly, in some embodiments of the methods of the invention, the stem cells are cultured in a multipotent, undifferentiated and proliferative state for at least 3, at least 4, at least 5, at least 6, at least 7, at least 8, at least 9, at least 10, at least 15, at least 20, at least 25, at least 30, at least 40, at least 50, at least 60, at least 70, at least 80, at least 90 or at least 100, passages under appropriate conditions.

A culture medium as disclosed herein may be capable of expanding at least 2, at least 3, at least 4, at least 5, at least 6, at least 7, at least 8, at least 9, or at least 10 different multipotent stem cell lines (e.g. different human ASC lines) in a multipotent, undifferentiated and proliferative state for multiple passages under appropriate conditions.

As noted elsewhere herein, the disclosure also provides a hermetically-sealed vessel containing a culture medium of the invention. Hermetically-sealed vessels may be preferred for transport or storage of the culture media, to prevent contamination. The vessel may be any suitable vessel, such as a bioreactor, a flask, a plate, a bottle, ajar, a vial or a bag.

As noted elsewhere herein, the disclosure also provides a method for preparing a culture medium, comprising the steps of: (a) obtaining a culture medium; and (b) adding a BCMA & TACI ligand and/or inducer thereof to the culture medium. Various different methods for preparing culture media are envisaged, depending on the specific ingredients to be included in the culture medium. For example, a method for preparing a culture medium may comprise the steps of: (a) obtaining a culture medium; and (b) adding BAFF or APRIL to the culture medium. In one embodiment, a method for preparing a culture medium may comprise the steps of: (a) obtaining a culture medium; and (b) adding BAFF and APRIL to the culture medium.

### Cell Culture Methods and Uses of the Culture Media

The culture media of the invention can be used to expand a population of multipotent stem cells. Accordingly, the invention provides the use of any culture medium as disclosed herein for expanding a population of multipotent stem cells.

The invention also provides an ex-vivo method for expanding a population of multipotent stem cells, comprising: (a) providing a population of multipotent stem cells; (b) providing a culture medium as disclosed herein; (c) contacting the stem cells with the culture medium; and (d) culturing the stem cells under appropriate conditions.

A method for 'expanding' a population of cells is one that involves increasing the number of stem cells in an initial population to generate an expanded population, whilst maintaining pluripotency and without significant differentiation, i.e. one that involves growth and division of stem cells, but not their differentiation.

The invention also provides a method of manufacture of a cellular therapy, comprising: (a) providing a population of multipotent stem cells; (b) providing a culture medium of the invention; (c) contacting the stem cell population with the culture medium; and (d) culturing the cells under appropriate conditions.

The methods of the invention may comprise culturing the cells in contact with a solid surface as described elsewhere herein. For example, the invention provides a method comprising: (a) providing a population of multipotent stem cells; (b) providing a culture medium as disclosed herein; (c) contacting the stem cells with the culture medium; and (d) culturing the cells under appropriate conditions and in contact with a solid surface. The invention also provides the use of a culture medium as disclosed herein and a solid surface to expand a population of multipotent stem cells. The multipotent stem cells may adhere, attach or be seeded onto said support. Typically, the cells are plated at a desired density such as between about 100 cells/cm² to about 100,000 cells/cm² (such as about 500 cells/cm² to about 50,000 cells/cm², or, more particularly, between about 1,000 cells/cm² to about 20,000 cells/cm²). If plated at lower densities (e.g., about 300 cells/cm²), the cells can be more easily clonally isolated. For example, after a few days, cells plated at such densities will proliferate into a homogeneous population. In a particular embodiment, the cell density is between 2,000-10,000 cells/cm².

The methods of the invention may comprise a step of passaging stem cells into a culture medium as disclosed herein. For example, the invention provides a method comprising: (a) providing a population of multipotent stem cells; (b) providing a culture medium as disclosed herein; (c) contacting the stem cells with the culture medium; (d) culturing the cells under appropriate conditions; (e) passaging the cells into a culture medium as disclosed herein; and (f) further culturing the cells under appropriate conditions.

It will be appreciated that the steps of the methods disclosed herein can be performed in any suitable order or at the same time, as appropriate, and need not be performed in the order in which they are listed. For example, in the above method the step of providing a population of multipotent stem cells may be performed before, after or at the same time as, the step of providing a culture medium.

After reaching the desired cell confluence, the cells can be expanded by means of consecutive passages. Cells may be passaged in the methods of the invention using known methods, e.g. by incubating the cells with trypsin and EDTA for between 5 seconds and 15 minutes at 37°C. A trypsin substitute (e.g. TrypLE from Invitrogen) may be used, if desired. Collagenase, dispase, accutase or other known reagents may also be used to passage the cells. Passaging is typically required every 2-8 days, such as every 4-7 days, depending on the initial seeding density. In some embodiments, the cell culture methods of the invention do not comprise any step of manually selecting undifferentiated cells when the cells are passaged. In some embodiments, the cell culture methods of the invention comprise automated passaging of the stem cells, i.e. without manipulation by a laboratory worker. The environment used to culture the stem cells may be sterile as well as temperature and pH stable.

The methods and uses of the invention may involve any culture medium or supplement as described herein. Accordingly, in some embodiments the methods of the invention may be serum and/or serum replacement-free methods. In some embodiments, the methods of the invention may be used to culture cells in the absence of contact with a layer of feeder cells.

It is preferred that said stem cell population is of adult origin, and it is further preferred that said cells are a mesenchymal stem cell population, most preferably adipose-derived stem cells.

Conditions for the culture of stem cells are known to the person skilled in the art. However it is particularly preferred that the culture is carried out in the presence of a solid support suitable for the adherence of mesenchymal stem cells.

Said method of manufacture may optionally further comprise the steps of: (e) passaging the cells into a culture medium as disclosed herein; and (f) further culturing the cells under appropriate conditions.

Thus, in a particular embodiment, cells are cultured without differentiation on a solid surface, usually made of a plastic material, such as Petri dishes or cell culture flasks, in the presence of a suitable cell culture medium [e.g., DMEM, typically supplemented with 5-15% (e.g., 10%) of a suitable serum, such as fetal bovine serum or human serum and a BCMA or TACI ligand such as BAFF or APRIL or substitutes thereof, and incubated under conditions which allow cells to adhere to the solid surface and proliferate. After incubation, cells are washed in order to remove non-adhered cells and cell fragments. The cells are maintained in culture in the same medium and under the same conditions until they reach the adequate confluence, typically, about 70%, about 80% or about 90% cell confluence, with replacement of the cell culture medium when necessary. After reaching the desired cell confluence, the cells can be expanded by means of consecutive passages using a detachment agent such as trypsin and seeding onto a new cell culture surface at an appropriate cell density (usually 2,000-10,000 cells/cm²). Thus, cells are then passaged at least two times in such medium without differentiating, while still retaining their developmental phenotype, and more preferably, the cells can be passaged at least 10 times (e.g., at least 15 times or even at least 20 times) without losing developmental phenotype. Typically, the cells are plated at a desired density such as between about 100 cells/cm² to about 100,000 cells/cm² (such as about 500 cells/cm² to about 50,000 cells/cm², or, more particularly, between about 1,000 cells/cm² to about 20,000 cells/cm²). If plated at lower densities (e.g., about 300 cells/cm²), the cells can be more easily clonally isolated. For example, after a few days, cells plated at such densities will proliferate into a homogeneous population. In a particular embodiment, the cell density is between 2,000-10,000 cells/cm².

Cells which remain adhered to the solid surface after such treatment comprising at least two passages are selected and the phenotype of interest is analyzed by conventional methods in order to confirm the identity of the ASC as will be mentioned below. Cells which remain adhered to the solid surface after the first passage are from heterogeneous origin; therefore, said cells must be subjected to at least another passage. As a result of the above method, a homogeneous cell population having the phenotype of interest is obtained. The adhesion of cells to the solid surface after at least two passages constitutes a preferred embodiment of the invention for selecting the ASC. Confirmation of the phenotype of interest can be carried out by using conventional means.

Preferably said expansion is carried out by duplication or triplication of said population at least 1, at least 2, at least 3, at least 4, at least 5, at least 10, at least 15 or at least 20 times. In a further embodiment said expansion is carried over at least 1, at least 2, at least 3, at least 4, at least 5, at least 10, at least 15 or at least 20 passages.

Cell-surface markers can be identified by any suitable conventional technique, usually based on a positive/negative selection; for example, monoclonal antibodies against cell-surface markers, whose presence/absence in the cells is to be confirmed, can be used; although other techniques can also be used. Thus, in a particular embodiment, monoclonal antibodies against one, two, three, four, five, six, seven of or preferably all of CD11b, CD11c, CD14, CD45, HLAII, CD31, CD34 and CD133 are used in order to confirm the absence of said markers in the selected cells; and monoclonal antibodies against one, two, three, four, of or preferably all of CD9, CD44, CD54, CD90 and CD 105 are used in order to confirm the presence thereof or detectable expression levels of, at least one of and preferably all of, said markers. Said monoclonal antibodies are known, commercially available or can be obtained by a skilled person in the art by conventional methods.

IFN-γ-inducible IDO activity in the selected cells can be determined by any suitable conventional assay. For example, the selected cells can be stimulated with IFN-γ and assayed for IDO expression; then conventional Western-blot analysis for IDO protein expression can be performed and IDO enzyme activity following IFN-γ stimulation of the selected cells can be measured by tryptophan-to-kynurenine conversion with for example via High Performance Liquid Chromatography (HPLC) analysis and photometric determination of kynurenine concentration in the supernatant as the readout. Since the ASC express IDO under certain conditions, any suitable technique which allows the detection of IDO activity following IFN-γ stimulation may be used for selecting the ASC. The amount of IDO produced depends on the number of cells per square centimetre, which is preferably at a level of 5000cells/cm² or more, but not limited to this concentration and the concentration of IFN-gamma, which ideally is 3ng/ml or more, but not limited to this concentration. The activity of IDO produced under the described conditions will result in a detectable production of kynurenine in the micro M range after 24hours or more.

The capacity of the selected cells to differentiate into at least two cell lineages can be assayed by conventional methods as known in the art.

ASC can be clonally expanded, if desired, using a suitable method for cloning cell populations. For example, a proliferated population of cells can be physically picked and seeded into a separate surface (or the well of a multi-well plate). Alternatively, the cells can be subcloned onto a multi-well plate at a statistical ratio for facilitating placing a single cell into each well (e.g., from about 0.1 to about 1 cell/well or even about 0.25 to about 0.5 cells/well, such as 0.5 cells/well). Of course, the cells can be cloned by plating them at low density (e.g., in a Petri dish or other suitable substrate) and isolating them from other cells using devices such as a cloning rings. The production of a clonal population can be expanded in any suitable culture medium. In any event, the isolated cells can be cultured to a suitable point when their developmental phenotype can be assessed.

It has been shown that *ex vivo* expansion of the ASC without inducing differentiation can be accomplished for extended time periods for example by using specially screened lots of suitable serum (such as fetal bovine serum or human serum). Methods for measuring viability and yield are known in the art (e. g., trypan blue exclusion).

Any of the steps and procedures for isolating the cells of the cell population of the invention can be performed manually, if desired. Alternatively, the process of isolating such cells can be facilitated and/or automated through one or more suitable devices, examples of which are known in the art.

The invention also provides the use of a composition comprising : (a) a culture medium according to the invention; and (b) stem cells for manufacturing a medicament for cellular therapy.

The invention also provides the use of a composition comprising : (a) a culture medium according to the invention; and (b) a solid surface for manufacturing a medicament for cellular therapy.

The methods of the invention may be performed using any suitable cell culture vessel as a support. Cell culture vessels of various shapes and sizes (e.g. flasks, single or multiwell plates, single or multiwell dishes, bottles, jars, vials, bags, bioreactors) and constructed from various different materials (e.g. plastic, glass) are known in the art. A suitable cell culture vessel can readily be selected by the skilled person.

### Culture Medium Supplements

Also disclosed herein is a culture medium supplement that can be used to produce a culture medium as disclosed herein. A "culture medium supplement' is a mixture of ingredients that cannot itself support multipotent stem cells, but which enables or improves multipotent stem cell culture when combined with other cell culture ingredients. The supplement can therefore be used to produce a functional cell culture medium of the invention by combining it with other cell culture ingredients to produce an appropriate medium formulation. The use of culture medium supplements is well known in the art. The disclosure provides a culture medium supplement that comprises a BCMA & TACI ligand and/or inducer thereof. The supplement may contain any ligands disclosed herein. The supplement may also contain one or more additional cell culture ingredients as disclosed herein, e.g. one or more cell culture ingredients selected from the group consisting of amino acids, vitamins, inorganic salts, carbon energy sources and buffers.

A culture medium supplement may be a concentrated liquid supplement (e.g. a 2x to 25Ox concentrated liquid supplement) or may be a dry supplement. Both liquid and dry supplements are well known in the art. A supplement may be lyophilized.

A supplement for use in the invention will typically be sterilized prior to use to prevent contamination, e.g. by ultraviolet light, heating, irradiation or filtration. A culture medium supplement may be frozen (e.g. at -20°C or -80°C) for storage or transport.

As noted elsewhere herein, the disclosure also provides a hermetically- sealed vessel containing a culture medium supplement of the invention. Hermetically-sealed vessels may be preferred for transport or storage of the culture media supplements disclosed herein, to prevent contamination. The vessel may be any suitable vessel, such as a bioreactor, a flask, a plate, a bottle, ajar, a vial or a bag.

### Compositions and Cell Culture Systems

The cell culture media and cell culture supplements disclosed herein can be used to expand a population of multipotent stem cells. Accordingly, the invention provides compositions arising during use of the cell culture media and cell culture supplements of the invention. For example, the invention provides a composition comprising: (a) a culture medium according to the invention; and (b) multipotent stem cells.

The compositions of the invention may be feeder cell-free compositions. A composition is conventionally considered to be feeder cell-free if the multipotent stem cells in the composition have been cultured for at least one passage in the absence of a feeder cell layer. A feeder cell-free composition of the invention will normally contain less than about 5%, less than about 4%, less than about 3%, less than about 2%, or less than about 1% feeder cells (expressed as a % of the total number of cells in the composition).

A solid surface may be used to provide a supportive function for the culture of adherent stem cells (for example but not limited to mesenchymal stem cells). The invention therefore also provides a composition containing: (a) a culture medium according to the invention; and (b) a solid surface. These compositions may further comprise multipotent stem cells.

A variety of substances have been used as surfaces for adherent stem cell culture, and an appropriate material can readily be selected by the skilled person. Preferably, the solid surface comprises plastic but may alternatively comprise of glass, extracellular matrix. The surface may be planar, tubular, or in the form of a scaffold, bead or fibre.

The invention also provides the use of (a) a solid surface; and (b) a BCMA & TACI ligand and/or inducer thereof, for expanding a population of multipotent stem cells. For example, the invention provides the use of BAFF and/or APRIL for expanding a population of multipotent stem cells. In one aspect the invention provides the use of BAFF or a BAFF substitute for expanding a population of multipotent stem cells. In an alternative aspect the invention provides the use of APRIL or an APRIL substitute for expanding a population of multipotent stem cells. In a further aspect the the invention provides the use of APRIL and BAFF or substitute thereof for expanding a population of multipotent stem cells.

The invention also provides a composition or cell culture vessel comprising (a) a solid surface; and (b) a BCMA & TACI ligand and/or inducer thereof, for expanding a population of multipotent stem cells. Accordingly in one aspect the invention provides a composition or cell culture vessel comprising BAFF or a BAFF substitute for expanding a population of multipotent stem cells. In an alternative aspect the invention provides a composition or cell culture vessel comprising APRIL or an APRIL substitute for expanding a population of multipotent stem cells. In a further aspect the the invention provides a composition or cell culture vessel comprising APRIL and BAFF or substitute thereof for expanding a population of multipotent stem cells.
The compositions of the invention may comprise serum, or may be serum-free and/or serum-replacement free, as described elsewhere herein.

The invention also provides a composition or cell culture vessel comprising (a) a solid surface; and (b) a culture medium of the present invention which has been supplemented with B27 Supplement and N2 Supplement.

### Multipotent stem cells

The culture media and methods disclosed herein are useful for expanding a population of multipotent stem cells, whilst maintaining the pluripotency of the cells and without problematic differentiation of the cells.

'Multipotent' stem cells are those that have the potential to differentiate into cells of all three germ layers (endoderm, mesoderm and ectoderm) under appropriate conditions.

Multipotent stem cells are not totipotent, i. e. they cannot form an entire organism, such as a foetus. Multipotent stem cells for use in the invention can be obtained using well-known methods (see below). It is envisaged that various types of multipotent stem cells may be used in conjunction with the invention, whether obtained from embryonic, foetal or adult tissue but are preferably derived from adult tissue sources.

The culture media disclosed herein may be used to culture mammalian stem cells, particularly human adult stem cells. Human adult stem cells that may be used in conjunction with the invention are preferably mesenchymal stem cells. Mouse or primate stem cells may also be used. In preferred embodiments, the stem cells are human adipose-derived stem cells (ASC).

Briefly, the ASC can be obtained by conventional means from any suitable source of connective tissue from any suitable animal as discussed above. Typically, human adipose cells are obtained from living donors, using well-recognized protocols such as surgical or suction lipectomy. Indeed, as liposuction procedures are so common, liposuction effluent is a particularly preferred source from which the ASC can be derived. Thus, in a particular embodiment, the ASC are from the stromal fraction of human adipose tissue obtained by liposuction.

The tissue is, preferably, washed before being processed to separate the ASC from the remainder of the material. In one commonly used protocol, the sample of tissue is washed with physiologically-compatible saline solution (e.g., phosphate buffered saline (PBS)) and then vigorously agitated and left to settle, a step that removes loose matter (e.g., damaged tissue, blood, erythrocytes, etc) from the tissue. Thus, the washing and settling steps are generally repeated until the supernatant is relatively clear of debris. The remaining cells generally will be present in clumps of various sizes, and the protocol proceeds using steps gauged to degrade the gross structure while minimizing damage to the cells themselves. One method of achieving this end is to treat the washed lumps of cells with an enzyme that weakens or destroys bonds between cells (e.g., collagenase, dispase, trypsin, etc.). The amount and duration of such enzymatic treatment will vary, depending on the conditions employed, but the use of such enzymes is generally known in the art. Alternatively, or in conjunction with such enzymatic treatment, the clumps of cells can be degraded using other treatments, such as mechanical agitation, sonic energy, thermal energy, etc. If degradation is accomplished by enzymatic methods, it is desirable to neutralize the enzyme following a suitable period, to minimize deleterious effects on the cells.

The degradation step typically produces a slurry or suspension of aggregated cells and a fluid fraction containing generally free stromal cells (e.g., red blood cells, smooth muscle cells, endothelial cells, fibroblast cells, and stem cells). The next stage in the separation process is to separate the aggregated cells from the ASC. This can be accomplished by centrifugation, which forces the cells into a pellet covered by a supernatant. The supernatant then can be discarded and the pellet suspended in a physiologically-compatible fluid. Moreover, the suspended cells typically include erythrocytes, and in most protocols it is desirable to lyse them. Methods for selectively lysing erythrocytes are known in the art, and any suitable protocol can be employed (e.g., incubation in a hyper -or hypotonic medium, by lysis using ammonium chloride, etc.). Of course, if the erythrocytes are lysed, the remaining cells should then be separated from the lysate, for example by filtration, sedimentation, or density fractionation.

Regardless of whether the erythrocytes are lysed, the suspended cells can be washed, recentrifuged, and resuspended one or more successive times to achieve greater purity. Alternatively, the cells can be separated on the basis of cell surface marker profile or on the basis of cell size and granularity.

Following the final isolation and re-suspension, the cells can be cultured and, if desired, assayed for number and viability to assess the yield. Preferably, the cells will be cultured without differentiation, on a solid surface, using a suitable cell culture media, at the appropriate cell densities and culture conditions.

A culture media as disclosed herein may be used to culture mesenchymal stem cells, particularly adipose-derived stem cells.

A culture media as disclosed herein may also be used to culture genetically modified multipotent stem cells. Genetically modified cells include those that have been transiently or stably modified by transformation, transfection, transduction, etc..

The culture media disclosed herein may also be used to culture cells that have been induced or transformed to form cells with stem cell-like properties. These multipotent stem cells may be genetically modified cells, such as mouse or human 'induced multipotent stem' (iPS) cells.

When a culture medium for use in the invention is used to expand a population of multipotent stem cells, the total number of undifferentiated, multipotent stem cells in the population will preferably increase at least 1.5 fold, at least 2 fold, at least 3 fold, at least 4 fold, at least 5 fold, at least 10 fold, at least 20 fold, at least 30 fold, at least 40 fold or at least 50 fold, between the time when a medium is applied to an initial cell population and the end of the culture period. It will be appreciated that the cells may be passaged one or more times during the culture period, after which the cells may be cultured in different cell culture vessels or cells may be discarded. If cells are cultured in different cell culture vessels after passaging, or if cells are discarded during passaging, this can be taken into account when calculating the fold difference in cell numbers obtained during a known culture period.

In preferred embodiments of the invention, at least 50%, at least 55%, at least 60%, at least 70%, at least 80%, at least 85%, at least 90%, at least 91%, at least 92%, at least 93%, at least 94% or at least 95%, of the stem cells (% by cell number) in an expanded population (z. e. the population after expansion of the initial population using a culture medium or method as disclosed herein) will be undifferentiated, multipotent and proliferative cells.

Methods for identifying undifferentiated, multipotent and proliferative stem cells, and for identifying the % of such cells in a population, are known and suitable methods for use with this invention can be selected by the skilled person depending on the stem cell type that is used.

Multipotent stem cells may be identified by their ability to differentiate into cells of all three germ layers e.g. by determining the ability of the cells to differentiate into cells showing detectable expression of markers specific for all three germ layers.

References in the singular (e.g. to "a cell" and equivalent references) encompass the plural (e.g. "cells") unless the context requires otherwise.

Various aspects and embodiments of the invention will now be described in more detail by way of example, with reference to specific culture media and methods. It will be appreciated that modification of detail may be made without departing from the scope of the invention. It is to be understood that the invention is not limited in its application to the embodiments set forth in the following Examples. The invention is capable of other embodiments or of being practiced or carried out in various ways.

### EXAMPLES

### Materials and Methods

### hASC isolation and cell culture

Human ASC were prepared from lipoaspirates of human adipose tissue from healthy adult donors. Briefly, human adipose tissue aspirates from healthy adult male and female donors were washed twice with phosphate-buffered saline (PBS) and digested with 0.075% collagenase (Type I, Invitrogen, Carlsbad, CA). The digested sample was washed with 10% fetal bovine serum (FBS), treated with 160 mM NH₄Cl to eliminate remaining erythrocytes and suspended in culture medium (Dulbecco's modified Eagle medium; DMEM) with 10% FBS). Cells were seeded (2-3 x 10⁴ cells/cm²) in tissue culture flasks and expanded (37°C, 5% CO₂), with change of culture medium every 3-4 days. Cells were transferred to a new flask (10³ cells/cm²) when they reached 90% confluence. For all experiments, pooled cells from independently expanded cultures from healthy donors (three male and three female; 35-47 years of age) were used. Cells were pooled and experiments performed at approximately the same population doubling (doubling 12-16).

Peripheral blood mononuclear cells (PBMC) were isolated from buffy coats using Ficoll-paque Plus (GE Healthcare Biosciences AB, Uppsala, Sweden) following the supplier's protocol. Briefly, blood samples were diluted with balanced salt solution and Ficoll was added to create a density gradient. After centrifugation, the interface containing mononuclear cells was collected. Purity was verified by flow cytometry. Buffy coats were provided by the National Transfusion Centre of the Comunidad Autónoma of Madrid, Spain.

### Recombinant proteins and antibodies

APRIL and BAFF recombinant proteins were purchased from R&D Systems (Minneapolis, MN), CXCL12 and IFN-γ were from Peprotech (London, UK); LPS (Escherichia coli 055:B5) was from Sigma Aldrich (St. Louis, MO) and poly I:C from InvivoGen (San Diego, CA). For flow cytometry, primary monoclonal antibodies to BCMA, TACI, HLA-I, HLA-II, CD80, CD86 and an isotype control were from BD Bioscience (San Jose, CA). Antibodies for western blotting were anti-phospho-Akt (Ser473), -Akt, -phospho-ERK1/2 (Thr202/Tyr204), -ERK1/2, -JNK (all from Cell Signaling Technology, Beverly, MA), phospho-JNK (Thr 183/Tyr185) from Invitrogen (Carlsbad, CA). Secondary antibodies were horseradish peroxidase-conjugated bovine anti-rabbit and goat anti-mouse IgG (Dako, Glostrup, Denmark)

### RT-PCR

Total RNA was isolated from cultured hASC or freshly purified PBMC (positive control) with TRI reagent (Sigma) and used (1-2 µg) for reverse transcription with the High Capacity cDNA Reverse Transcription Kit (Applied Biosystems). Semiquantitative PCR with cDNA serial dilution (1:0, 1:5, 1:25) was performed using the 5'PRIME Master Mix Kit with a thermocycler (iCycler; BioRad). β-actin served as internal control. Primer sequences were as follows: APRIL forward 5'-AAGGGTATCCCTGGCAGAGT-3' (SEQ ID NO: 5) and reverse 5'-GCAGGACAGAGTGCTGCTT-3' (SEQ ID NO: 6), BAFF forward 5'-ACTGAAAATCTTTGAACCACCAG-3' (SEQ ID NO: 7) and reverse 5'-TTGCAAGCAG TCTTGAGTGAC-3' (SEQ ID NO: 8), TACI forward 5'-CAACTCGGGAAGGTACCAAGGATT-3' (SEQ ID NO: 9) and reverse 5'-CGCCACCAGGAAGCAGCAGAGGAC-3' (SEQ ID NO: 10), BCMA forward 5'-TTTTCGTGCTAATGTTTTTGCTAA-3' (SEQ ID NO: 11) and reverse 5'-TTCATCACCAGTCCTGCTCTTTTC-3' (SEQ ID NO: 12), BAFF-R forward 5'-CTGGTCCTGGTGGGTCTG-3' (SEQ ID NO: 13) and reverse 5'-ACCTTGTCCAGGGGCTCT-3' (SEQ ID NO: 14), beta-actin forward 5'-CCCAGCACAATGAAGATCAA-3' (SEQ ID NO: 15) and reverse 5'-CGATCCACACGGAGTACTTG-3' (SEQ ID NO: 16) (all from Sigma).

### Flow Cytometry

hASCs were harvested, stained with appropriate fluorochrome-coupled antibodies and measured by flow cytometry (BD FACS Calibur). Data were anayzed using the FlowJo program (TreeStar, Ashland, OR).

### Cell Viability and Proliferation

Cell viability was assessed by the CellTiter AQueous One Solution Cell Proliferation Assay (Promega, Madison, WI) according to manufacturer's protocols. hASC (3.5 x 10³ cells/well) were cultured for 6 days in 96-well plates with 100 ng/ml APRIL or BAFF. Absorbance (OD 490 nm) was measured in an ELISA plate reader (n = at least three experiments). Values were normalized to the absorbance of cells cultured without stimuli (100%). Additionally, cell proliferation was measured by a colorimetric BrdU incorporation assay. hASC were cultured in p60 plates with 100 ng/ml APRIL or BAFF; BrdU (20 µM) was added for the last three days. At day 6, BrdU incorporation was detected by flow cytometry using an anti-BrdU antibody. Three independent MTS and BrdU assays were performed and triplicates included for each condition.

### Immunosuppression assay

For CFSE labeling, freshly purified PBMC (10-20 x 106) were washed extensively to remove FBS, resuspended in 200 µl of a 10 µM CFSE solution (Sigma), and incubated with constant shaking (37°C, 10 min). Cells were washed and cultured overnight, and one aliquot was used to set up and control FL-1 voltage for CFSE in the cytometer. After overnight resting, CFSE-labeled PBMC were activated with the Pan T Cell Activation Kit (microbeads coated with anti-CD3, -CD2 and -CD28, Miltenyi Biotech) following manufacturer's instructions, or were left unactivated. Two days before PBMC activation, hASC were plated in a 24-well plate (4 x 10⁴ cells/well). PBMC (10⁶ cells/ml) were cultured alone or with hASC, with or without BAFF or APRIL (100 ng/ml). At day 5, PBMC were harvested and cell proliferation was determined according to loss of CFSE in the FL-1 channel. Data were analyzed using Cellquest-Pro software on gated lymphocytes (based on forward scatter/side scatter properties). The percentage of proliferating lymphocytes was calculated by gating in the region (M1) of the FL-1 channel corresponding to the last 48 h of culture (three generations). CaliBRITE beads (BD Bioscience) were used prior to each assay to calibrate the cytometer.

### Activation and Signaling

hASC were stimulated with 100 ng/ml APRIL or BAFF (10, 30, 60, 120 min, 37°C), placed on ice, washed with ice-cold PBS, and lysed with cell lysis buffer (Roche, Indianapolis, IN). Extracts were quantified by absorbance, resolved in SDS-PAGE and analyzed by immunoblot. Antibodies used were anti-phospho-Akt (Ser473), -Akt, - phospho-ERK1/2 (Thr202/Tyr204), and -ERK1/2) (BD Bioscience); secondary antibodies were horseradish peroxidase-conjugated bovine anti-rabbit and goat anti-mouse IgG.

### Cytokine detection

hASC cells were cultured with 100 ng/ml APRIL, BAFF or no stimulus and then stimulated with 1 µg/ml LPS or poly I:C. IL-6 and IL-8 concentration in the medium after 48 h, was measured by the Cytometric Bead Array (CBA) immunoassay (BD Bioscience) following manufacturer instructions. Data were acquired using a FACScalibur cytometer and analyzed using FCAP array software (BD Bioscience). Alternatively, hASC cells were cultured with medium or IFNγ 10, 30 or 100 ng/ml; or CXCL12 10, 30 or 100 nM. Supernatants were collected five days later. APRIL and BAFF concentrations in the medium were determined by ELISA (R&D Systems for BAFF, Bender MedSystem for APRIL).

### Western blotting

Plated hASC (1.5 x 104 cells/cm2) were stimulated with APRIL or BAFF (100 ng/ml) and lysed with lysis buffer (Roche) with protease and phosphatase inhibitors. Proteins in extracts were quantified by absorbance with the BCA protein assay kit (Pierce), and equal amounts of protein homogenate resolved in SDS-PAGE, and transferred to PVDF membranes (Millipore). Blots were blocked in TBS-T containing 5% BSA and probed with anti-phospho-Akt (Ser473), -Akt, -phospho-ERK1/2 (Thr202/Tyr204), and - ERK1/2 antibodies (all from Cell Signaling Technology). Membranes were washed in TBS-T, incubated with HRP-conjugated secondary antibodies (Dako), and reactivity detected with ECL (GE Healthcare).

### Statistics

Statistical analysis (Student's t-test) was performed using GraphPad Prism 4.0 (GraphPad Software). Results are expressed as mean ± SD. P values (2-tailed) *** p<0.0001, ** p<0.001 and * p<0.001 were considered statistically significant.

### Results

### Expression of APRIL, BAFF and their receptors in hASC

To study the expression profile of the APRIL/BAFF system by RT-PCR, mRNA samples from cultured hASC were prepared and analyzed using the above-mentioned human primers. mRNA transcripts were detected for both ligands, for BCMA and TACI, receptors shared by APRIL and BAFF, and for BAFF-R, which binds exclusively to BAFF (Fig. 1A). PBMC purified from buffy coats were used as a positive control, as they express all the members of the family; APRIL and BAFF are produced by monocytes and TACI, BCMA and BAFF-R by B cells and plasma cells. Analysis of hASC supernatants showed an APRIL protein concentration of 3.97 ± 0.72 ng/ml (n = 6, day 6), whereas BAFF was undetectable (Fig 1B).

BCMA and TACI expression were confirmed by flow cytometry (Fig. 1C), which did not detect BAFF-R, probably due to the antibody or staining procedure used. The results show that the APRIL/BAFF system, comprised of two ligands and three receptors, is present in hASC and that in vitro cultured hASC secrete APRIL to the medium while BAFF remains undetectable.

### APRIL and BAFF activate ERK and PI3K transduction pathways

Human ASC were stimulated with APRIL or BAFF, and the phosphorylation kinetics of MAP kinases (p38, ERK1/2, JNK), Akt, and IκBα was analyzed by Western blot. Both APRIL and BAFF induced rapid phosphorylation of Akt and ERK1/2, with stronger activation for Akt than for ERK1/2 (Fig. 2; no clear effect on JNK, IκBα or p38). Western blot bands suggested that BAFF promotes a greater, more sustained response than APRIL, probably due to synergy of the BAFF-specific receptor, BAFF-R, with TACI and/or BCMA signaling. These data indicate that APRIL and BAFF ligands are able to signal through their receptors in hASC by activating the kinase proteins Akt and ERK1/2.

### APRIL and BAFF enhance hASC proliferation in a ERK-dependent manner

In an MTS assay, both cytokines enhanced hASC basal growth after 6 days in culture, although BAFF was a stronger stimulator than APRIL (140% and 114% increase, respectively, considering basal growth 100%; Fig. 3A). The greater effect of BAFF compared to APRIL might be attributed to its binding to the BAFF-R. Cell proliferation by BrdU incorporation in APRIL- or BAFF-stimulated hASC was measured and similar increases in the percentage of BrdU-positive cells was observed, from 13% (control) to 29.4% (BAFF) and 27.5% (APRIL) (Fig. 3B). It was tested whether Akt or ERK participates in the mechanism underlying APRIL/BAFF-increased hASC proliferation, using inhibitors of PI3K (LY294002) and ERK activation (U0126) to block these pathways. The MTS assay showed a decrease in basal hASC growth from 100% (control) to 60.3% when PI3K but not ERK inhibitor was added to medium (Fig. 3C), indicating involvement of the PI3K transduction pathway in basal growth of hASC. APRIL and BAFF did not promote cell growth when ERK but not PI3K was inhibited, implying that both TNF ligands induce this effect through ERK signaling (Fig. 3C).

### APRIL and BAFF do not alter hASC immunological properties

APRIL and BAFF are overexpressed in serum and tissue lesions of patients with chronic inflammatory diseases such as RA or SLE. It is therefore of interest, from a clinical and therapeutic point of view, to understand whether BAFF and APRIL can modulate immunological features of hASC. Since APRIL/BAFF are described as costimulators with the TLR (Toll-like receptor) ligands, and their secretion is induced by TLR activation, it was tested whether this is the case in hASC. Cells were cultured alone or with APRIL or BAFF, then activated with LPS or poly I:C. Neither ligand induced APRIL or BAFF secretion, nor did APRIL or BAFF affect LPS- or poly I:C-induced IL-6 or IL-8 production (Fig. 4A). The immunomodulatory capacity of hASC is a key factor in their potential for therapeutic use. The effect of BAFF/APRIL on the ability of hASC to suppress immune responses was tested by analyzing proliferation of activated CFSE-labeled PBMC alone or in the presence of allogeneic hASC cultured with medium alone or supplemented with BAFF or APRIL (Fig. 4B). As predicted, hASC suppressed PBMC proliferation efficiently. Addition of APRIL or BAFF to medium did not alter the ability of hASC to suppress PBMC proliferation. BAFF/APRIL activation did not induce indoleamine 2,3-dioxygenase (IDO), a tryptophan-catabolizing enzyme involved in the suppression of lymphocyte proliferation. To test whether APRIL or BAFF activation modify the immunogenic profile of hASC, HLA-I, HLA-II, CD80 and CD86 expression was analyzed by flow cytometry, and found that BAFF and APRIL do not affect hASC expression of these activation molecules. These results indicate that stimulation with APRIL or BAFF does not markedly impair the immunogenic and immunomodulatory properties of hASC.

### APRIL and BAFF secretion in hASC is differentially induced by IFN-γ and CXCL12

Molecules that might promote APRIL or BAFF secretion in hASC were evaluated, focusing on IFN-γ and CXCL12, two cytokines implicated in hASC-mediated immunosuppression and cell migration, respectively; Fig. 4C shows a dose response curve measuring APRIL and BAFF concentrations following stimulation with IFN-γ (10, 30, 100 ng/ml) or CXCL12 (10, 30, 100 nM). These results demonstrate that CXCL12 preferentially enhances APRIL, whereas IFN-γ promotes BAFF secretion, indicating that each TNF cytokine is secreted by hASC in response to different stimuli.

## Claims

1. The use of a culture medium for expanding a population of mesenchymal stem cells, wherein the mesenchymal stem cells are derived from adipose tissue and wherein said medium comprises a BCMA & TACI ligand, wherein the BCMA & TACI ligand is selected from the group consisting of:
(i) a polypeptide that shows at least 85% identity to the polypeptide of SEQ ID NO:1,
(ii) a soluble polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a soluble polypeptide that shows at least 85% identity to the extracellular domain of the polypeptide of SEQ ID NO:2.

2. The use of a culture medium according to claim 1, which comprises between about 10 pM and about 100 mM of a BCMA & TACI ligand.

3. A composition comprising: (a) a culture medium; and (b) mesenchymal stem cells wherein the mesenchymal stem cells are derived from adipose tissue and wherein the culture medium comprises a BCMA & TACI ligand, wherein the BCMA & TACI ligand is selected from the group consisting of:
(i) a polypeptide that shows at least 85% identity to the polypeptide of SEQ ID NO:1,
(ii) a soluble polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a a soluble polypeptide that shows at least 85% identity to the extracellular domain of the polypeptide of SEQ ID NO:2.

4. A method for expanding a population of mesenchymal stem cells wherein the mesenchymal stem cells are derived from adipose tissue, comprising: (a) providing a population of mesenchymal stem cells; (b) providing a culture medium; (c) contacting the mesenchymal stem cells with the culture medium; and (d) culturing the stem cells under appropriate conditions; said medium comprises a BCMA & TACI ligand, wherein the BCMA & TACI ligand is selected from the group consisting of:
(i) a polypeptide that shows at least 85% identity to the polypeptide of SEQ ID NO:1,
(ii) a soluble polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a a soluble polypeptide that shows at least 85% identity to the extracellular domain of the polypeptide of SEQ ID NO:2.

5. The use of a BCMA & TACI ligand in the manufacture of a mesenchymal stem cell therapy medicament, wherein the mesenchymal stem cells are derived from adipose tissue and wherein the BCMA & TACI ligand is selected from the group consisting of:
(i) a polypeptide that shows at least 85% identity to the polypeptide of SEQ ID NO:1,
(ii) a soluble polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a a soluble polypeptide that shows at least 85% identity to the extracellular domain of the polypeptide of SEQ ID NO:2.

6. A BCMA & TACI ligand for use as a mesenchymal stem cell therapy medicament, wherein the mesenchymal stem cells are derived from adipose tissue and wherein the BCMA & TACI ligand is selected from the group consisting of:
(i) a polypeptide that shows at least 85% identity to the polypeptide of SEQ ID NO:1,
(ii) a soluble polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a a soluble polypeptide that shows at least 85% identity to the extracellular domain of the polypeptide of SEQ ID NO:2.

7. The use of (a) a solid surface; and (b) a BCMA & TACI ligand, in a method for expanding a population of mesenchymal stem cells wherein the mesenchymal stem cells are derived from adipose tissue and wherein the BCMA & TACI ligand is selected from the group consisting of:
(i) a polypeptide that shows at least 85% identity to the polypeptide of SEQ ID NO:1,
(ii) a soluble polypeptide that shows at least 85% identity to a polypeptide comprising all of the TNF-like domain of the polypeptide of SEQ ID NO:1,
(iii) a polypeptide that shows at least 85% identity to the polypeptide of SEQ ID NO:2 and
(iv) a a soluble polypeptide that shows at least 85% identity to the extracellular domain of the polypeptide of SEQ ID NO:2.

8. The composition according to claim 3 wherein said ligand comprises BAFF and/or APRIL.

9. The method according to claim 4 wherein said ligand comprises BAFF and/or APRIL.

10. The use according to any of claims 1, 2, 5 or 7 wherein said ligand comprises BAFF and/or APRIL.

11. The BCMA & TACI ligand for use according to claim 6 wherein said ligand comprises BAFF and/or APRIL.

## Patentansprüche

1. Verwendung eines Kulturmediums zum Expandieren einer Population von mesenchymalen Stammzellen, wobei die mesenchymalen Stammzellen aus adipösem Gewebe stammen, und, wobei das Medium einen BCMA- und TACI-Liganden enthält, wobei der BCMA- & TACI-Ligand aus der Gruppe ausgewählt wird, welche besteht aus:
(i) einem Polypeptid, welches wenigstens 85% Identität zu dem Polypeptid SEQ ID NO:1 zeigt,
(ii) einem löslichen Polypeptid, welches 85% Identität zu einem Polypeptid zeigt, welches alle der TNF-artigen Domäne des Polypeptids SEQ ID NO:1 enthält,
(iii) einem Polypeptid, welches wenigstens 85% Identität zu dem Polypeptid SEQ ID NO:2 zeigt, und
(iv) einem löslichen Peptid, welches wenigstens 85% Identität zu der extrazellulären Domäne des Polypeptids SEQ ID NO:2 zeigt.

2. Verwendung eines Kulturmediums nach Anspruch 1, welches zwischen ungefähr 10 pM und ungefähr 100 mM eines BCMA- und TACI-Liganden enthält.

3. Zusammensetzung enthaltend: (a) ein Kulturmedium und (b) mesenchymale Stammzellen, wobei die mesenchymalen Stammzellen aus adipösem Gewebe stammen, und, wobei das Kulturmedium einen BCMA- und TACI-Liganden enthält, wobei der BCMA- & TACI-Ligand aus der Gruppe ausgewählt ist, welche besteht aus:
(i) einem Polypeptid, welches wenigstens 85% Identität zu dem Polypeptid SEQ ID NO:1 zeigt,
(ii) einem löslichen Polypeptid, welches 85% Identität zu einem Polypeptid zeigt, welches alle der TNF-artigen Domäne des Polypeptids SEQ ID NO:1 enthält,
(iii) einem Polypeptid, welches wenigstens 85% Identität zu dem Polypeptid SEQ ID NO:2 zeigt, und
(iv) einem löslichen Peptid, welches wenigstens 85% Identität zu der extrazellulären Domäne des Polypeptids SEQ ID NO:2 zeigt.

4. Verfahren zum Expandieren einer Population von mesenchymalen Stammzellen, wobei die mesenchymalen Stammzellen aus adipösem Gewebe stammen, wobei das Verfahren umfasst: (a) Bereitstellen einer Population von mesenchymalen Stammzellen; (b) Bereitstellen eines Kulturmediums; (c) Kontaktieren der mesenchymalen Stammzellen mit dem Kulturmedium und (d) Kultivieren der Stammzellen unter geeigneten Bedingungen, wobei das Medium einen BCMA- und TACI-Liganden enthält, wobei der BCMA- und TACI-Ligand aus der Gruppe ausgewählt ist, welche besteht aus:
(i) einem Polypeptid, welches wenigstens 85% Identität zu dem Polypeptid SEQ ID NO:1 zeigt,
(ii) einem löslichen Polypeptid, welches 85% Identität zu einem Polypeptid zeigt, welches alle der TNF-artigen Domäne des Polypeptids SEQ ID NO:1 enthält,
(iii) einem Polypeptid, welches wenigstens 85% Identität zu dem Polypeptid SEQ ID NO:2 zeigt, und
(iv) einem löslichen Peptid, welches wenigstens 85% Identität zu der extrazellulären Domäne des Polypeptids SEQ ID NO:2 zeigt.

5. Verwendung eines BCMA- und TACI-Liganden bei der Herstellung eines mesenchymalen Stammzellentherapiemedikaments, wobei die mesenchymalen Stammzellen aus adipösem Gewebe stammen, und, wobei der BCMA- und TACI-Ligand aus der Gruppe ausgewählt ist, welche besteht aus:
(i) einem Polypeptid, welches wenigstens 85% Identität zu dem Polypeptid SEQ ID NO:1 zeigt,
(ii) einem löslichen Polypeptid, welches 85% Identität zu einem Polypeptid zeigt, welches alle der TNF-artigen Domäne des Polypeptids SEQ ID NO:1 enthält,
(iii) einem Polypeptid, welches wenigstens 85% Identität zu dem Polypeptid SEQ ID NO:2 zeigt, und
(iv) einem löslichen Peptid, welches wenigstens 85% Identität zu der extrazellulären Domäne des Polypeptids SEQ ID NO:2 zeigt.

6. BCMA- und TACI-Ligand zur Verwendung als ein mesenchymales Stammzellentherapiemedikament, wobei die mesenchymalen Stammzellen aus adipösem Gewebe stammen, und, wobei der BCMA- und TACI-Ligand aus der Gruppe ausgewählt ist, welche besteht aus:
(i) einem Polypeptid, welches wenigstens 85% Identität zu dem Polypeptid SEQ ID NO:1 zeigt,
(ii) einem löslichen Polypeptid, welches 85% Identität zu einem Polypeptid zeigt, welches alle der TNF-artigen Domäne des Polypeptids SEQ ID NO:1 enthält,
(iii) einem Polypeptid, welches wenigstens 85% Identität zu dem Polypeptid SEQ ID NO:2 zeigt, und
(iv) einem löslichen Peptid, welches wenigstens 85% Identität zu der extrazellulären Domäne des Polypeptids SEQ ID NO:2 zeigt.

7. Verwendung einer (a) festen Oberfläche und (b) eines BCMA- und TACI-Liganden in einem Verfahren zum Expandieren einer Population von mesenchymalen Stammzellen, wobei die mesenchymalen Stammzellen aus adipösem Gewebe stammen, und, wobei der BCMA- und TACI-Ligand aus der Gruppe ausgewählt wird, welche besteht aus:
(i) einem Polypeptid, welches wenigstens 85% Identität zu dem Polypeptid SEQ ID NO:1 zeigt,
(ii) einem löslichen Polypeptid, welches 85% Identität zu einem Polypeptid zeigt, welches alle der TNF-artigen Domäne des Polypeptids SEQ ID NO:1 enthält,
(iii) einem Polypeptid, welches wenigstens 85% Identität zu dem Polypeptid SEQ ID NO:2 zeigt, und
(iv) einem löslichen Peptid, welches wenigstens 85% Identität zu der extrazellulären Domäne des Polypeptids SEQ ID NO:2 zeigt.

8. Zusammensetzung nach Anspruch 3, wobei der Ligand BAFF und/oder APRIL enthält.

9. Verfahren nach Anspruch 4, wobei der Ligand BAFF und/oder APRIL enthält.

10. Verwendung nach einem der Ansprüche 1, 2, 5 oder 7, wobei der Ligand BAFF und/oder APRIL enthält.

11. BCMA- und TACI-Ligand zur Verwendung nach Anspruch 6, wobei der Ligand BAFF und/oder APRIL enthält.

## Revendications

1. Utilisation d'un milieu de culture pour amplifier une population de cellules souches mésenchymateuses, dans laquelle les cellules souches mésenchymateuses sont issues d'un tissu adipeux et dans laquelle ledit milieu comporte un ligand BCMA & TACI, dans laquelle le ligand BCMA & TACI est choisi parmi le groupe constitué de :
(i) un polypeptide qui présente au moins 85 % d'identité avec le polypeptide de la SEQ ID N° : 1,
(ii) un polypeptide soluble qui présente au moins 85 % d'identité avec un polypeptide comportant la totalité du domaine analogue du TNF du polypeptide de la SEQ ID N° : 1,
(iii) un polypeptide qui présente au moins 85 % d'identité avec le polypeptide de la SEQ ID N° : 2 et
(iv) un polypeptide soluble qui présente au moins 85 % d'identité avec le domaine extracellulaire du polypeptide de la SEQ ID N° : 2.

2. Utilisation d'un milieu de culture selon la revendication 1, qui comporte entre environ 10 pM et environ 100 mM d'un ligand BCMA & TACI.

3. Composition comportant : (a) un milieu de culture, et (b) des cellules souches mésenchymateuses dans laquelle les cellules souches mésenchymateuses sont issues d'un tissu adipeux et dans laquelle le milieu de culture comporte un ligand BCMA & TACI, dans laquelle le ligand BCMA & TACI est choisi parmi le groupe constitué de :
(i) un polypeptide qui présente au moins 85 % d'identité avec le polypeptide de la SEQ ID N° : 1,
(ii) un polypeptide soluble qui présente au moins 85 % d'identité avec un polypeptide comportant la totalité du domaine analogue du TNF du polypeptide de la SEQ ID N° : 1,
(iii) un polypeptide qui présente au moins 85 % d'identité avec le polypeptide de la SEQ ID N° : 2 et
(iv) un polypeptide soluble qui présente au moins 85 % d'identité avec le domaine extracellulaire du polypeptide de la SEQ ID N° : 2.

4. Procédé pour amplifier une population de cellules souches mésenchymateuses, dans lequel les cellules souches mésenchymateuses sont issues d'un tissu adipeux, comportant les étapes consistant à : (a) fournir une population de cellules souches mésenchymateuses, (b) fournir un milieu de culture, (c) mettre en contact les cellules souches mésenchymateuses avec le milieu de culture, et (d) mettre en culture les cellules souches dans des conditions appropriées, ledit milieu comportant un ligand BCMA & TACI, dans lequel le ligand BCMA & TACI est choisi parmi le groupe constitué de :
(i) un polypeptide qui présente au moins 85 % d'identité avec le polypeptide de la SEQ ID N° : 1,
(ii) un polypeptide soluble qui présente au moins 85 % d'identité avec un polypeptide comportant la totalité du domaine analogue du TNF du polypeptide de la SEQ ID N° : 1,
(iii) un polypeptide qui présente au moins 85 % d'identité avec le polypeptide de la SEQ ID N° : 2 et
(iv) un polypeptide soluble qui présente au moins 85 % d'identité avec le domaine extracellulaire du polypeptide de la SEQ ID N° : 2.

5. Utilisation d'un ligand BCMA & TACI dans la fabrication d'un médicament thérapeutique à base de cellules souches mésenchymateuses, dans laquelle les cellules souches mésenchymateuses sont issues d'un tissu adipeux et dans laquelle le ligand BCMA & TACI est choisi parmi le groupe constitué de :
(i) un polypeptide qui présente au moins 85 % d'identité avec le polypeptide de la SEQ ID N° : 1,
(ii) un polypeptide soluble qui présente au moins 85 % d'identité avec un polypeptide comportant la totalité du domaine analogue du TNF du polypeptide de la SEQ ID N° : 1,
(iii) un polypeptide qui présente au moins 85 % d'identité avec le polypeptide de la SEQ ID N° : 2 et
(iv) un polypeptide soluble qui présente au moins 85 % d'identité avec le domaine extracellulaire du polypeptide de la SEQ ID N° : 2.

6. Ligand BCMA & TACI pour une utilisation en tant que médicament thérapeutique à base de cellules souches mésenchymateuses, dans lequel les cellules souches mésenchymateuses sont issues d'un tissu adipeux et dans lequel le ligand BCMA & TACI est choisi parmi le groupe constitué de :
(i) un polypeptide qui présente au moins 85 % d'identité avec le polypeptide de la SEQ ID N° : 1,
(ii) un polypeptide soluble qui présente au moins 85 % d'identité avec un polypeptide comportant la totalité du domaine analogue du TNF du polypeptide de la SEQ ID N° : 1,
(iii) un polypeptide qui présente au moins 85 % d'identité avec le polypeptide de la SEQ ID N° : 2 et
(iv) un polypeptide soluble qui présente au moins 85 % d'identité avec le domaine extracellulaire du polypeptide de la SEQ ID N° : 2.

7. Utilisation (a) d'une surface solide, et (b) d'un ligand BCMA & TACI, dans un procédé pour amplifier une population de cellules souches mésenchymateuses, dans laquelle les cellules souches mésenchymateuses sont issues d'un tissu adipeux et dans laquelle le ligand BCMA & TACI est choisi parmi le groupe constitué de :
(i) un polypeptide qui présente au moins 85 % d'identité avec le polypeptide de la SEQ ID N° : 1,
(ii) un polypeptide soluble qui présente au moins 85 % d'identité avec un polypeptide comportant la totalité du domaine analogue du TNF du polypeptide de la SEQ ID N° : 1,
(iii) un polypeptide qui présente au moins 85 % d'identité avec le polypeptide de la SEQ ID N° : 2 et
(iv) un polypeptide soluble qui présente au moins 85 % d'identité avec le domaine extracellulaire du polypeptide de la SEQ ID N° : 2.

8. Composition selon la revendication 3, dans laquelle ledit ligand comporte BAFF et/ou APRIL.

9. Procédé selon la revendication 4, dans lequel ledit ligand comporte BAFF et/ou APRIL.

10. Utilisation selon l'une quelconque des revendications 1, 2, 5 ou 7, dans laquelle ledit ligand comporte BAFF et/ou APRIL.

11. Ligand BCMA & TACI destiné à une utilisation selon la revendication 6, dans lequel ledit ligand comporte BAFF et/ou APRIL.
